(19) Europäisches Patentamt
European Patent Office
Office européen des brevets

(11) EP 3 329 252 B1

(12) **FASCICULE DE BREVET EUROPEEN**

(45) Date de publication et mention de la délivrance du brevet:
**06.05.2020 Bulletin 2020/19**

(21) Numéro de dépôt: **16750657.5**

(22) Date de dépôt: **26.07.2016**

(51) Int Cl.:
***G01N 21/3577*** *(2014.01)* ***G01N 33/28*** *(2006.01)*

(86) Numéro de dépôt international:
**PCT/EP2016/067743**

(87) Numéro de publication internationale:
**WO 2017/017082 (02.02.2017 Gazette 2017/05)**

(54) **PROCÉDÉ DE DÉTERMINATION EN LIGNE D'UN INDICE DE BASICITÉ D'UN CORPS LIQUIDE ET UTILISATION DE CE PROCÉDÉ POUR UN LUBRIFIANT**

VERFAHREN ZUR ONLINE-BESTIMMUNG EINER BASENZAHL EINER FLÜSSIGEN SUBSTANZ UND VERWENDUNG DIESES VERFAHRENS FÜR EIN SCHMIERMITTEL

METHOD FOR ONLINE DETERMINATION OF A BASE NUMBER OF A LIQUID BODY AND USE OF THIS METHOD FOR A LUBRICANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.07.2015 FR 1557138**

(43) Date de publication de la demande:
**06.06.2018 Bulletin 2018/23**

(73) Titulaire: **WIKA Tech S.A.S.**
**73370 Le Bourget-du-Lac (FR)**

(72) Inventeurs:
- **ADJALI, Mustapha**
  **73100 Aix Les Bains (FR)**
- **JUSTON, Raphael**
  **73000 Chambéry (FR)**
- **CHAUDOREILLE, François**
  **73100 Gresy Sur Aix (FR)**

(74) Mandataire: **Lavoix**
**62, rue de Bonnel**
**69448 Lyon Cedex 03 (FR)**

(56) Documents cités:
**WO-A1-2013/186338** **WO-A2-03/073075**
**CN-B- 101 782 512** **DE-A1- 19 650 397**

- **AL-GHOUTI M A ET AL: "Application of chemometrics and FTIR for determination of viscosity index and base number of motor oils", TALANTA, ELSEVIER, AMSTERDAM, NL, vol. 81, no. 3, 6 février 2010 (2010-02-06), pages 1096-1101, XP026966421, ISSN: 0039-9140 [extrait le 2010-02-06]**
- **Craig Winterfield ET AL: "Automated Acid and Base Number Determination of Mineral-Based Lubricants by Fourier Transform Infrared Spectroscopy: Commercial Laboratory Evaluation", Journal of Laboratory Automation Society for Laboratory Automation and Screening, 30 septembre 2014 (2014-09-30), pages 577-586, XP055281259, DOI: 10.1177/2211068214551825 Extrait de l'Internet: URL:http://jla.sagepub.com/content/19/6/57 7.full.pdf [extrait le 2016-06-16]**
- **L. BEN MOHAMMADI ET AL: "A low cost mid-infrared sensor for on line contamination monitoring of lubricating oils in marine engines", OPTICAL SENSING II, vol. 7726, 30 avril 2010 (2010-04-30), pages 77260M-1, XP055280857, 1000 20th St. Bellingham WA 98225-6705 USA ISSN: 0277-786X, DOI: 10.1117/12.854476 ISBN: 978-1-62841-971-9 cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 329 252 B1

## Description

La présente invention a trait à un procédé de détermination en ligne d'un indice de basicité d'un corps liquide, notamment un lubrifiant. La présente invention trouve une application particulière pour la détermination de la basicité d'un lubrifiant circulant dans un équipement, tel qu'un moteur de navire.

Dans le domaine des moteurs à combustion interne utilisés sur les navires de commerce, il est connu qu'il convient de suivre la situation d'un moteur en analysant un lubrifiant circulant dans ce moteur. Une telle analyse permet de détecter des phénomènes d'usure ou de corrosion qui ont tendance à se produire dans un moteur. Par le passé, le fonctionnement des moteurs était relativement stabilisé et il suffisait de contrôler la qualité d'un lubrifiant de façon ponctuelle, lors des escales, pour anticiper les opérations de maintenance à réaliser. De nos jours, les moteurs sont de plus en plus élaborés et sensibles aux phénomènes d'usure ou de corrosion, de sorte que des analyses doivent être réalisées en mer, notamment pour suivre l'indice de basicité ou BN (de l'Anglais « Base Number ») de l'huile moteur. Ceci impose de former le personnel et d'embarquer un matériel élaboré, dont le fonctionnement est relativement difficile à maîtriser, même par un matelot formé. En outre, ceci augmente la charge de travail du personnel.

Dans ce cadre, il est connu de l'article « A low cost mid-infrared sensor for on line contamination monitoring of lubricating oils in marine engines » de Ben Mohammadi et al. (Optical Sensing and Détection Conférence - Bruxelles - 12-15/4/2010) de prévoir un système d'analyse du TBN (ou « Total Base Number ») qui correspond à l'indice de basicité total, au moyen d'un capteur dans lequel est disposé un échantillon du lubrifiant à étudier. Le matériel utilisé est élaboré et sa manipulation est complexe, au point qu'il ne pourrait que difficilement être embarqué sur un navire. En outre, cette approche requiert, de la part de l'utilisateur, une bonne connaissance des phénomènes mesurés, au point qu'elle n'est pas forcément à la portée d'un matelot. Ce matériel utilise un modèle qui n'est adapté que pour un seul type d'huile. Il devient donc très difficile de l'intégrer dans un process où les liquides utilisés sont amenés à changer en permanence.

WO-A-03/073075 divulgue une méthode d'analyse de la basicité d'un lubrifiant au cours de laquelle une mesure, effectuée sur un échantillon d'un lubrifiant à contrôler, est comparée à des mesures effectuées sur des échantillons de référence. La méthode envisagée dans ce document est basée sur l'utilisation de l'absorbance du lubrifiant qui est relativement peu précise dans la mesure où elle est sensible au bruit des informations collectées. En effet, les valeurs erratiques ou de « bruit » risquent d'être considérées comme aussi pertinentes que les valeurs significatives mesurées. Cette technique impose de faire un « blanc » avant chaque mesure, ce qui est contraignant et génère une perte de temps. En outre, il est nécessaire de procéder au nettoyage du capteur après chaque mesure car un résidu de l'échantillon précédent altérerait la mesure en cours. Enfin, le modèle obtenu avec cette méthode est valable pour un seuil type de lubrifiant et fortement dépendant de la précision de lecture de l'absorbance.

Il est également connu de WO-A-2013/186338 de déterminer un indice de basicité globale d'un lubrifiant par une analyse FTIR d'un spectre d'absorption. Cette méthode est complexe à mettre en œuvre.

De façon générale, des problèmes comparables se posent lorsqu'il convient de déterminer l'indice de basicité d'un corps liquide.

C'est à ces inconvénients qu'entend plus particulièrement remédier l'invention en proposant un nouveau procédé de détermination d'un indice de basicité d'un corps liquide qui est fiable et moins dépendant du bruit dans les données collectées, tout en étant facile à mettre en œuvre pour un opérateur.

A cet effet, l'invention concerne un procédé de détermination en ligne d'un indice de basicité d'un corps liquide, à caractériser, notamment un lubrifiant, par spectroscopie moyenne infra rouge, dans lequel on met en œuvre au moins une étape a consistant à détecter l'intensité d'un signal infra rouge passant à travers un échantillon du corps liquide à caractériser. Conformément à l'invention, ce procédé comprend également au moins des étapes supplémentaires consistant à :

b) postérieurement à l'étape **a,** calculer la valeur de transmittance d'ondes infra rouge à travers l'échantillon pour p nombres d'onde, avec p un entier naturel supérieur ou égal à deux ;

c) exprimer l'indice de basicité du corps liquide à caractériser sous la forme

$$BN = Tr.M_{(px1)}+R$$

où

| | |
|---|---|
| BN | est l'indice de basicité du lubrifiant à caractériser, |
| Tr | est un jeu de valeurs de transmittance calculées à l'étape b |
| $M_{(px1)}$ | est un ensemble de données de modèle contenant des coefficients déterminés à partir de valeurs d'indice de basicité mesurées et de valeurs de transmittance mesurées pour des corps liquides de référence, |
| R | est un résidu de modèle, déterminé à partir des valeurs mesurées d'indice de basicité et de transmittance. |

Grâce à l'invention, l'indice de basicité peut être automatiquement calculé à l'étape **c** en se basant sur les valeurs de transmittance détectées à l'étape **b** et sur le modèle préalablement établi, sans que l'utilisateur ait à consulter des tables de données difficiles à lire et qui peuvent le conduire à faire des erreurs d'interprétation. En particulier, les étapes **b** et **c** peuvent être automatisées, ce qui permet d'envisager la mise en œuvre du procédé de l'invention à bord d'un navire, sous le seul contrôle d'un matelot. L'invention peut également être mise en œuvre pour la détermination de l'indice de basicité d'un autre liquide utilisé dans d'autres circonstances.

Selon des aspects avantageux mais non obligatoires de l'invention, un tel procédé peut incorporer une ou plusieurs des caractéristiques suivantes, prises dans toute combinaison techniquement admissible :

- Lors de l'étape b), la valeur de transmittance pour chaque nombre d'onde est calculée comme le rapport de l'intensité du signal infra rouge passant à travers l'échantillon sur l'intensité à vide du signal.
- En variante, l'étape b) comprend des étapes élémentaires b1) et b2) consistant pour b1) à calculer, pour chaque nombre d'onde, le rapport de l'intensité du signal infra rouge passant à travers l'échantillon sur l'intensité à vide du signal, et pour b2) à calculer la valeur de transmittance sous la forme d'une valeur corrigée du rapport calculé à l'étape b1). Dans ce cas on peut prévoir que, lors de l'étape élémentaire b2), la valeur du rapport est corrigée par filtrage et/ou retrait d'une ligne de base.
- La valeur de transmittance comprend une matrice unidirectionnelle à une ligne et p colonnes et l'ensemble de données du modèle est une matrice unidirectionnelle à p lignes et une colonne.
- Le procédé comprend des étapes préalables, mises en œuvre pour la détermination du modèle et consistant à :

  $\alpha$1) détecter l'intensité $I_0(N)$ d'un signal infra rouge, pour les p nombres d'onde, dans le vide,
  $\alpha$2) détecter l'intensité I(N) d'un signal infra rouge, pour les p nombres d'onde, au sein de n échantillons, chacun représentatif d'un corps liquide de référence ;
  $\alpha$3) calculer la transmittance de chaque corps liquide de référence comme un ensemble de valeurs définies, pour chaque nombre d'onde, comme le rapport de l'intensité détectée à l'étape $\alpha$2 sur l'intensité détectée à l'étape $\alpha$1;
  $\alpha$4) créer un premier ensemble de données de références comprenant les valeurs de transmittance calculées à l'étape $\alpha$3 pour les n échantillons et pour les p nombres d'onde ;
  $\alpha$5) mesurer l'indice de basicité de chaque corps liquide de référence ;
  $\alpha$6) créer un deuxième ensemble de données de référence comprenant les n valeurs d'indice de basicité mesurées à l'étape $\alpha$5 ; et
  $\alpha$7) déterminer par calcul l'ensemble de données et le résidu du modèle, sur la base des premier et deuxième ensembles de données de référence.

- Lors de l'étape $\alpha$3), la transmittance de chaque corps liquide de référence est calculée comme un ensemble de valeurs définies, pour chaque nombre d'onde, comme le rapport de l'intensité détectée à l'étape $\alpha$2) sur l'intensité détectée à l'étape $\alpha$1).
- En variante, l'étape $\alpha$3) comprend des étapes élémentaires $\alpha$31) et $\alpha$32) consistant pour $\alpha$31) à calculer, pour chaque nombre d'ondes, le rapport de l'intensité détectée à l'étape $\alpha$2) sur l'intensité détectée à l'étape $\alpha$1), et pour $\alpha$32) à calculer la valeur de transmittance sous la forme d'une valeur corrigée du rapport calculé à l'étape $\alpha$31), de préférence par filtrage et/ou retrait d'une ligne de base.
- Le premier ensemble de données de référence est une matrice bi-dimensionnelle à n lignes et p colonnes, chaque ligne comprenant des valeurs de transmittance calculées à l'étape $\alpha$3 pour un corps liquide de référence et p nombres d'onde et chaque colonne comprenant des valeurs de transmittance calculées pour un nombre d'onde et les n corps liquide de référence, alors que le deuxième ensemble de données de référence est une matrice unidirectionnelle à n lignes et une colonne..
- Le procédé comprend des étapes préalables supplémentaires, mises en œuvre au cours de l'étape $\alpha$7 pour la détermination du modèle et consistant à :

  $\beta$1) définir un premier ensemble données de calcul qui est une image du premier ensemble de données de référence ;
  $\beta$2) définir un deuxième ensemble de données de calcul qui est une image du deuxième ensemble de données de référence ;
  $\beta$3) définir une première variable dépendante du premier ensemble de données de calcul selon la relation : T = X.W

    où T est la première variable,
    X est le premier ensemble de données de calcul,

W est un premier coefficient de pondération,

β4) définir une deuxième variable dépendante du deuxième ensemble de données de calcul selon la relation : C = Y.U

où C est la deuxième variable,
Y est le deuxième ensemble de données de calcul,
U est un deuxième coefficient de pondération, et

β5) calculer de façon itérative, les valeurs des deux coefficients de pondération, en maximisant la covariance des premier et deuxième variables.

- Le procédé comprend des étapes préalables supplémentaires mises en œuvre au cours de l'étape α7 pour la détermination du modèle et consistant à :

β6) appliquer, à chaque pas itératif, une régression linéaire du premier ensemble de données de calcul sur la première variable ;
β7) appliquer, à chaque pas itératif, une régression linéaire du deuxième ensemble de données de calcul sur la première variable ;
β8) calculer, à chaque pas itératif, une valeur estimée du premier ensemble de données de calcul, en appliquant une transposée d'une pente de la régression linéaire, déterminée à ce pas, à la première variable calculée à ce pas.
β9) calculer, à chaque pas itératif, une valeur réelle du premier ensemble de données de calcul, en soustrayant la valeur estimée calculée à l'étape β8 d'une valeur réelle de ce premier ensemble de données de calcul calculée au pas itératif précédent.
β10) calculer, à chaque pas itératif de l'étape β5, une valeur estimée du deuxième ensemble de données de calcul, en appliquant une transposée d'une pente de la régression linéaire, déterminée à ce pas, à la première variable calculée à ce pas.
β11) calculer, à chaque pas itératif, une valeur réelle du deuxième ensemble de données de calcul, en soustrayant la valeur estimée calculée à l'étape β10 d'une valeur réelle de ce deuxième ensemble de données de calcul calculée au pas itératif précédent.

- Les étapes β5 à β11 sont répétées jusqu'à ce que la valeur du deuxième ensemble de données devienne inférieure à une valeur de seuil prédéterminée
- La première variable est une matrice bi-dimensionnelle à n lignes et p colonnes, la deuxième variable est une matrice unidirectionnelle à n lignes et une colonne, le premier coefficient de pondération est une matrice bi-dimensionnelle à p lignes et p colonnes, le deuxième coefficient de pondération est un nombre, alors que, à chaque pas itératif, les coefficients de pondération sont calculés selon les équations :

$$W_k = {}^tX_{k-1}\, Y_{k-1}\, {}^tY_{k-1}\, X_{k-1}$$

$$U_k = {}^tY_{k-1}\, X_{k-1}\, {}^tX_{k-1}\, Y_{k-1}$$

où l'indice k appliqué à une matrice indique la valeur de cette matrice au pas d'itération k, que la régression linéaire de l'étape β6 est effectuée sur la base d'une équation de type :

$$X_{k-1} = T_k\, {}^tp_k + X_{k_k}$$

où $p_k$ est une matrice de coefficients de régression déterminée par la méthode des moindres carrés et qui s'exprime sous la forme :

$$p_k = \frac{{}^tX_{k-1}\, T_k}{{}^tT_k T_k}$$

que la régression linéaire de l'étape β7 est effectuée sur la base d'une équation de type :

$$Y_{k-1} = T_k{}^t q_k + Y_{k_k}$$

où $q_k$ est une matrice de coefficients de régression déterminée par la méthode des moindres carrés et qui s'exprime sous la forme :

$$q_k = \frac{{}^tY_{k-1}\,T_k}{{}^tT_k T_k}$$

- Le procédé comprend des étapes préalables supplémentaires γ1 et γ2, mises en œuvre pour la détermination du modèle et consistant à exprimer le deuxième ensemble de données de référence en fonction de la première variable selon la relation :

$$Y^0 = \sum_1^K W_k{}^t q_k + Y_K$$

où K est le nombre d'itérations de l'étape β5), k est un entier naturel entre 1 et K, Tk est la première variable au rang d'itération k,
${}^tq_k$ est la transposée de la pente de régression linéaire utilisée à l'étape β10)
$Y_K$ est le deuxième ensemble de données de calcul au rang d'itération K, et déterminer l'ensemble de données du modèle et le résidu comme étant respectivement égaux à $M_{(px1)} = \sum_1^K W_k{}^t q_k$ et R = $Y_K$, où $W_k$ est le coefficient de pondération au rang d'itération k.

L'invention concerne également l'utilisation d'un procédé tel que mentionné ci-dessus pour déterminer l'indice de basicité d'un lubrifiant circulant dans un équipement d'un navire, en particulier dans un moteur de navire.
L'invention sera mieux comprise et d'autres avantages de celle-ci apparaîtront plus clairement à la lumière de la description qui va suivre, de deux modes de réalisation d'un procédé conforme à son principe, donnée uniquement à titre d'exemple et faite en référence aux dessins annexés dans lesquels :

- la figure 1 est une représentation schématique de principe d'une installation permettant de mettre en œuvre le procédé de l'invention ;
- la figure 2 est un schéma bloc d'un premier procédé selon l'invention ;
- la figure 3 est un schéma bloc de certaines étapes préalables, utiles au procédé de la figure 2 ;
- la figure 4 est un schéma bloc de certaines autres étapes préalables, utiles au procédé de la figure 2 ;
- la figure 5 est un schéma bloc analogue à la figure 2, pour un deuxième procédé selon l'invention et ;
- la figure 6 est un schéma bloc analogue à la figure 3, pour le deuxième procédé selon l'invention.

L'installation 2 représentée à la figure 1 comprend un réservoir 4 dans lequel est stocké un échantillon E d'un lubrifiant L de moteur de propulsion de navire et qui forme une chambre d'analyse dont le volume interne peut être relativement faible, notamment inférieur à 1 $cm^3$. En pratique, le volume interne du réservoir 4 peut être inférieur à 0,1 $cm^3$. Le réservoir 4 est alimenté avec une quantité de lubrifiant destinée à constituer l'échantillon E, soit en déversant à la main une quantité de lubrifiant L destinée à constituer l'échantillon, soit à partir d'un piquage branché sur une conduite d'évacuation du lubrifiant, en sortie du moteur du navire. Une vanne 6 montée sur une conduite 8 de purge du réservoir 4 permet de contrôler l'évacuation de la quantité de lubrifiant présente dans le réservoir 4. La vanne 6 est une électrovanne commandée par une unité électronique 10 configurée à cet effet.
L'installation 2 comprend également un émetteur 12 et un récepteur 14 également commandés par l'unité électronique de commande 10. En particulier, le récepteur 14 est capable de délivrer à l'unité électronique de commande 10 un signal électrique S14 représentatif d'un signal infra rouge qu'il détecte.
L'émetteur 12 et le récepteur 14 sont configurés pour travailler ensemble dans la gamme moyenne infra rouge. L'émetteur 12 émet des ondes dans la gamme moyenne infra rouge, c'est-à-dire avec un nombre d'ondes N compris entre 4000 $cm^{-1}$ et 600 $cm^{-1}$. Le capteur 14 est en fait un spectroscope qui peut travailler sur la plage de nombres d'ondes précités. En pratique, pour la détermination de l'indice basicité d'un lubrifiant de moteur de navire, l'émetteur 12 et le récepteur 14 travaillent dans une gamme de nombres d'onde N comprise entre environ 1900 $cm^{-1}$ et environ 900 $cm^{-1}$. Ceci tient compte du fait que la réponse aux rayonnements infrarouges de différents traceurs de l'indice de basicité, notamment les carbonates de calcium ou de magnésium, les sulfonates, se trouve dans cette gamme.

Lorsqu'un échantillon E de lubrifiant est présent dans le réservoir 4, il est possible d'émettre avec l'émetteur 12 un signal en forme de faisceau F de lumière infra rouge qui est détecté par le récepteur 14 après avoir traversé les parois du récipient 4 et l'échantillon E de lubrifiant. La méthode de la présente invention repose sur la détermination de la transmittance d'un échantillon E de lubrifiant L placé dans le réservoir 4, cette transmittance étant utilisée pour déterminer l'indice de basicité BN de ce lubrifiant. La transmittance Tr d'un liquide est définie comme le rapport $I/I_0$ où I est l'intensité du faisceau F détectée par le récepteur 14 lorsque ce faisceau F traverse à la fois le réservoir 4 et l'échantillon E de ce liquide et $I_0$ est la même intensité lorsque le faisceau F avec le réservoir 4 vide d'échantillon, donc contenant de l'air, ou avec le réservoir retiré. Dans ce cas où le réservoir est vide d'échantillon ou retiré, on parle de mesure « à vide », car il n'y a pas d'échantillon présent dans l'installation 2. En pratique, la transmittance $T_r$ est mesurée pour un liquide avec différentes valeurs de nombre d'onde N du faisceau F, sur la gamme 1900 $cm^{-1}$ - 900 $cm^{-1}$ citée précédemment.

**Calcul du l'indice de basicité**

Au début de l'utilisation de l'installation 2, on met en œuvre une étape préalable z au cours de laquelle on détermine l'intensité $I_0$ (N) du faisceau F capté par le récepteur 14 pour différents nombres d'ondes N, lorsque le réservoir 4 est retiré ou vide d'échantillon. Dans cet exemple, cette détermination a lieu pour 128 nombres d'ondes N. On crée ainsi un ensemble de 128 valeurs d'intensité à vide $I_0$ (N) pour les différentes valeurs de nombres d'ondes N. Ceci est représenté par l'étape z à la figure 2.

Cette étape z est mise en œuvre une seule fois, même si plusieurs échantillons E sont ensuite étudiés au moyen des étapes décrites ci-après. Ainsi, il n'est pas nécessaire de faire un « blanc » avec l'installation 2 avant chaque mesure.

Le nombre p de nombres d'ondes N pour lesquels on effectue la mesure peut être choisi différent de 128, par exemple égal à 64 ou 256, en fonction de la précision recherchée. Ce nombre est supérieur ou égal à 2.

Lorsqu'il convient de déterminer en ligne l'indice de basicité d'un lubrifiant L, on introduit une quantité de ce lubrifiant dans le réservoir 4 pour constituer l'échantillon E, au sein de la ligne de mesure représentée à la figure 1 comme représenté à la figure 1 et on mesure l'intensité I(N) du faisceau F détectée par le récepteur 14, lorsque ce faisceau F a traversé à la fois le réservoir 4 et l'échantillon E de lubrifiant L pour chacun des p nombres d'ondes. Ceci est effectué au cours d'une première étape a de la méthode d'invention.

On calcule alors, pour chaque nombre d'ondes N, la valeur de la transmittance Tr(N) comme étant égale au rapport $I(N)//I_0(N)$ pour ce nombre d'ondes. Ceci a lieu lors d'une étape b et permet de construire le spectre de transmittance Tr de l'échantillon mesuré avec l'installation 2, sous la forme d'une matrice à une ligne et p colonnes qui peut s'écrire comme suit :

$$Tr_{(1\times p)} = [tr_1 \quad tr_2 \cdots \quad tr_p] \qquad \text{(équation 1)}$$

où p est le nombre de nombres d'ondes pris en compte, ce nombre étant supérieur ou égal à 2, comme expliqué ci-dessus, et par exemple égal à 128.

Lorsque cette matrice de transmittance Tr est connue, on utilise, dans une étape c ultérieure du procédé de l'invention, un modèle calculé en laboratoire et qui comprend une matrice unidirectionnelle $M_{(px1)}$ à p lignes et une colonne. Cette matrice $M_{(px1)}$ est, quant à elle, constituée de valeurs $m_i$ de pondération pour les p nombres d'onde où i est un entier naturel entre 1 et p. Elle s'exprime selon la relation suivante :

$$M_{(p\times 1)} = \begin{bmatrix} m_1 \\ \vdots \\ m_p \end{bmatrix} \qquad \text{(équation 2)}$$

La méthode de calcul de ces valeurs de pondérations $m_i$ est décrite ci-après, dans la partie « calcul du modèle ».

L'indice de basicité du lubrifiant L, souvent dénommé BN, comme « base number », ou TBN, comme « total base number », est calculé dans cette étape c par la relation matricielle suivante :

$$BN = Tr.M_{(px1)} + R \qquad \text{(équation 3)}$$

où

BN est l'indice de basicité,
Tr est le spectre de transmittance de l'échantillon,
$M_{(px1)}$ est la matrice du modèle et
R est un résidu, c'est-à-dire une erreur acceptée du modèle, qui s'exprime sous la forme d'un nombre.

Ainsi, la méthode de l'invention, qui utilise l'équation 3 pour calculer l'indice de basicité BN, peut être automatisée, notamment réalisée dans l'unité 10, sans que l'utilisateur doive avoir une compétence particulière et ne passe une longue période à étudier des tables de référence, difficiles à lire.

En d'autres termes, les étapes **b** et **c** mentionnées ci-dessus peuvent être mises en œuvre de façon automatique une fois que l'échantillon E de lubrifiant est en place dans le réservoir 4, ce qui permet de calculer l'indice de basicité de façon rapide et fiable et d'afficher une valeur directement lisible par l'utilisateur, voire de transmettre automatiquement cette valeur à un organe de pilotage du moteur dont est extrait le lubrifiant L. En d'autres termes, toutes les étapes de calcul sont intégrées et le capteur ne renvoie que la valeur finale de l'indice de basicité via un protocole de communication industriel spécifique. Aucune intervention ou manipulation extérieure n'est nécessaire. Dans l'étape a) mentionnée ci-dessus, la vanne 6 est fermée et l'intensité I(N) du faisceau F est mesurée alors que le liquide est au repos dans le réservoir 4. En variante, cette mesure peut avoir lieu « au défilé », c'est-à-dire lorsque le liquide s'écoule dans le réservoir 4, qui fonctionne alors comme un tronçon de conduite, tandis que la vanne 6 est ouverte.

**Calcul du modèle**

Le calcul du modèle a lieu préalablement à la mise en œuvre des étapes a à c mentionnées ci-dessus et vise à déterminer la matrice $M_{(px1)}$ et le résidu R. Une fois ce modèle calculé, il peut être utilisé pour la détermination de l'indice de basicité de plusieurs échantillons, au moyen des étapes a à c, comme expliqué ci-dessus.

Pour calculer le modèle, on procède à partir de n échantillons de lubrifiant dont l'indice de basicité est mesurable par une technique connue, n étant un nombre entier supérieur ou égal à 2.

Dans un premier temps, on procède à l'acquisition des spectres en transmittance de ces n échantillons au moyen de l'émetteur 12 et du capteur ou récepteur 14. Pour ce faire, on met en œuvre une première étape $\alpha 1$ au cours de laquelle on détecte l'intensité du signal formé par le faisceau F à vide, lorsque le réservoir 4 est retiré ou présent mais vide d'échantillon. Cette étape $\alpha 1$ est la même que l'étape **z** mentionnée ci-dessus au sujet de la définition de la transmittance. En pratique, l'étape $\alpha 1$ peut être mise en œuvre au cours du calcul du modèle et son résultat peut être utilisé lors de l'étape **b** mentionnée ci-dessus. L'étape **z** mentionnée ci-dessus est donc facultative.

On met également en œuvre une deuxième étape $\alpha 2$ au cours de laquelle on mesure l'intensité du signal formé par le faisceau F à travers chacun des n échantillons et du réservoir 4. Cette étape $\alpha 2$ est du même type que l'étape **a,** sauf qu'elle est effectuée avec les n échantillons de référence. Après mise en œuvre des étapes $\alpha 1$ et $\alpha 2$, l'unité 10 met automatiquement en œuvre une étape $\alpha 3$ au cours de laquelle est calculée la transmittance Tr de chacun des n échantillons pour chacun des p nombres d'onde considérés.

Ceci permet de générer, dans une étape suivante $\alpha 4$ un premier ensemble de données de référence $X^0$ qui est construit sous la forme d'une matrice à n lignes et p colonnes qui s'exprime sous la forme :

$$X^0_{(n\times p)} = \begin{bmatrix} x^0_{11} & x^0_{12} & \cdots & x^0_{1p} \\ x^0_{21} & x^0_{22} & \cdots & x^0_{2p} \\ \vdots & \vdots & & \vdots \\ x^0_{n1} & x^0_{n2} & \cdots & x^0_{np} \end{bmatrix}, \qquad \text{(équation 4)}$$

Dans cette matrice, chaque ligne donne les valeurs de transmittance d'un échantillon pour les p nombres d'onde et chaque colonne donne les valeurs de transmittance des n échantillons pour un nombre d'onde.

Dans un deuxième temps, au cours d'une étape $\alpha 5$, une analyse en laboratoire est effectuée pour ces n échantillons. L'indice de basicité de chacun de ces échantillons est mesuré par la méthode ASTM D2896. En variante, une autre méthode de mesure de l'indice de basicité peut être mise en œuvre, par exemple un titrage potentiométrique ASTMD4739, ou un titrage par indicateur colorimétrique ASTM D5984 ou ASTM D974

Cette mesure de l'étape $\alpha 5$ permet de générer, dans une étape suivante $\alpha 6$ un deuxième ensemble de données

de référence $Y^0$ qui est construit sous la forme d'une matrice unidirectionnelle à n lignes et 1 colonne et qui s'exprime sous la forme :

$$Y^0_{(n\times1)} = \begin{bmatrix} y^0_1 \\ \vdots \\ y^0_n \end{bmatrix} \qquad \text{(équation 5)}$$

En variante, les étapes $\alpha5$ et $\alpha6$ sont mises en œuvre avant les étapes $\alpha1$ à $\alpha4$, ou en même temps.

A partir des ensembles de données de référence $X^0$ et $Y^0$, le procédé calcule, dans une étape $\alpha7$ les valeurs de la matrice $M_{(px1)}$ et du résidu R. Cette étape $\alpha7$ est représentée à la figure 3 avec les étapes $\alpha1$ à $\alpha6$ et constitue avec ces étapes la partie préliminaire du procédé de l'invention. L'étape $\alpha7$ est représentée de façon détaillée à la figure 4.

Au cours de cette étape $\alpha7$ et à partir des ensembles $X^0$ et $Y^0$ de données de référence, on peut construire, respectivement dans deux étapes $\beta1$ et $\beta2$, deux ensembles de données de calcul, qui sont respectivement des images des ensembles $X^0$ et $Y^0$, sous la forme de variables matricielles X et Y définies comme suit :

$$X_{(n\times p)} = \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix}, \qquad \text{(équation 6)}$$

$$Y_{(n\times1)} = \begin{bmatrix} y_1 \\ \vdots \\ y_n \end{bmatrix} \qquad \text{(équation 7)}$$

où $X_{(n\times p)}$ est une matrice représentant n spectres des valeurs de transmittance à p nombres d'onde et $Y_{(n\times1)}$ est une matrice représentant des valeurs de d'indice de basicité BN correspondant aux n échantillons. X est une variable d'entrée et Y est une variable de sortie pour l'équation 3, puisque Y correspond aux valeurs d'indice de basicité ou BN.

On souhaite pouvoir exploiter l'équation 3, en écrivant la variable de sortie Y correspondant aux valeurs d'indice de basicité BN des n échantillons, en fonction de la variable d'entrée X correspondant aux spectres en transmittance associés.

Le but du calcul est donc d'arriver à écrire :

$$Y = X.B + \varepsilon \qquad \text{(équation 8)}$$

où B est une matrice à p lignes et une colonne et $\varepsilon$ est un résidu.

En d'autres termes, le but est de trouver une relation entre ces deux grandeurs X et Y qui s'exprime comme la relation de l'équation 3 que l'on souhaite utiliser dans l'étape c. Ceci permet de considérer que la matrice B de l'équation 8 est égale la matrice M et que la valeur $\varepsilon$ est égale au résidu R. L'écriture de l'équation 8 vise donc à déterminer la matrice $M_{(px1)}$ du modèle et son résidu R.

Pour cela, on crée de nouvelles variables T et C en procédant à une transformation linéaire des grandeurs X et Y, respectivement au cours d'une étape $\beta3$ et d'une étape $\beta4$, sous la forme :

$$T = X.W \qquad \text{(équation 9)}$$

et

$$C = Y.U \qquad \text{(équation 10)}$$

où W et U sont des coefficients de pondération pour les variables X et Y.

La transformation des grandeurs X et Y lors des étapes $\beta3$ et $\beta4$ est effectuée pour maximiser la covariance entre les nouvelles variables créées T et C dans le but de trouver la corrélation entre les grandeurs X et Y telle que décrite dans l'équation 8. La covariance Cov(T,C) des variables T et C s'exprime sous la forme suivante :

$$Cov\ (T,C) = Cov(XW,YU) = \frac{1}{n-1}\,{}^tW\,{}^tX\ Y\ U \qquad \text{(équation 11)}$$

En d'autres termes, on cherche à calculer les coefficients de pondération W et U qui maximisent l'équation 11.

La résolution de ce problème se fait par une méthode itérative, en considérant des valeurs successives $W_k$ et $U_k$ des coefficients de pondération, où k est le pas d'itération. Elle consiste en les étapes suivantes qui seront répétées *K* fois, K étant un nombre entier supérieur ou égal à 2 :

Initialement, on pose $X=X^0$ et $Y=Y^0$.

Dans une étape β5, on calcule $W_k$ et de $U_k$ pour k compris entre 1 et K.

D'après Agnar Höskuldsson (PLS regression methods. Journal of chemometrics, 2 :221-228, 1988), les solutions $W_k$ et $U_k$ de l'équation 11 ont la forme suivante :

$$W_k = {}^tX_{k-1}\,Y_{k-1}\,{}^tY_{k-1}\,X_{k-1} \qquad \text{(équation 12)}$$

et

$$U_k = {}^tY_{k-1}\,X_{k-1}\,{}^tX_{k-1}\,Y_{k-1} \qquad \text{(équation 13)}$$

Ceci permet de calculer $T_k$ et $C_k$ comme suit :

$$T_k = X_{k-1}W_k \qquad \text{(équation 14)}$$

et

$$C_k = Y_{k-1}U_k \qquad \text{(équation 15)}$$

On procède ensuite, respectivement lors d'une étape β6 et lors d'une étape β7, à une régression de $X_{k-1}$ sur $T_k$ et $C_k$, dans le but d'arriver à estimer $X_{k-1}$ et $Y_{k-1}$ avec les nouvelles variables $T_k$ et $C_k$, et plus particulièrement estimer $X_{k-1}$ et $Y_{k-1}$ en fonction $T_k$ selon les relations :

$$X_{k-1} = T_k\,{}^tp_k + X_{k_k} \qquad \text{(équation 16)}$$

et

$$Y_{k-1} = T_k\,{}^tq_k + Y_{k_k} \qquad \text{(équation 17)}$$

La régression sur $C_k$ ne sert pas pour le calcul du modèle en lui-même mais pour évaluer ses performances.

On calcule donc, lors de ces étapes β6 et β7, des coefficients $p_k$ et $q_k$ de régression correspondant à la régression de $X_k$ et $Y_k$ respectivement sur $T_k$, par la méthode des moindres carrés. Ces coefficients de régression $p_k$ et $q_k$ sont des matrices à p lignes et p colonnes. On obtient :

$$p_k = \frac{{}^tX_{k-1}\,T_k}{{}^tT_kT_k} \qquad \text{(équation 16')}$$

et

$$q_k = \frac{{}^tY_{k-1}\,T_k}{{}^tT_kT_k} \qquad \text{(équation 17')}$$

En variante, une méthode de régression autre que celle des moindres carrés peut être utilisée. En particulier, on peut faire une estimation par la méthode du maximum de vraisemblance ou encore par la méthode d'inférence bayésienne.

Lors d'une étape suivante β8, on utilise le résultat de la régression pour calculer une valeur estimée $X'_k$ de la grandeur X au rang k d'itération, selon la relation suivante :

$$X'_k = T_k{}^t p_k \quad \text{(équation 18)}$$

De la même façon, lors d'une étape suivante β9 on calcule une valeur estimée Y'k de la grandeur Y au rang k d'itération, selon la relation suivante :

$$Y'_k = T_k{}^t q_k \quad \text{(équation 19)}$$

A partir des équations 18 et 19, on déduit, dans deux étapes ultérieures β10 et β11, la valeur réelle $X_k$ et $Y_k$ des grandeurs X et Y au rang d'itération k, grâce aux équations suivantes :

$$X_k = X_{k-1} - X'_k = X_{k-1} - T_k{}^t p_k \quad \text{(équation 20)}$$

où $X_{k-1}$ est la valeur réelle calculée au rang d'itération k-1 et $X'_k$ ou $T_k{}^t p_k$ est la valeur estimée au rang d'itération k, et

$$Y_k = Y_{k-1} - Y'_k = Y_{k-1} - T_k{}^t q_k \quad \text{(équation 21)}$$

où $Y_{k-1}$ est la valeur réelle calculée au rang d'itération k-1 et $Y'_k$ ou $T_k{}^t q_k$ est la valeur estimée au rang d'itération k.

Il vient donc l'écriture de $X_{k-1}$ et $Y_{k-1}$ en fonction de la nouvelle variable $T_k$, soit :

$$X_{k-1} = T_k{}^t p_k + X_k \quad \text{(équation 22)}$$

et

$$Y_{k-1} = T_k{}^t q_k + Y_k \quad \text{(équation 23)}$$

On incrémente ensuite la valeur de k puis on réitère les étapes β5 à β11, en prenant comme valeurs initiales pour chaque pas d'itération k+1, les valeurs $X_k$ et $Y_k$ obtenues, lors du pas d'itération, précédent k par les équations 20 et 21.

Ces étapes de calcul β5 à β11 sont réitérées K fois jusqu'à obtenir une corrélation satisfaisante entre les valeurs de sortie mesurées du modèle $Y^0_{(n\times1)}$ et les valeurs d'entrée correspondant aux mesures effectuées $X^0_{(n\times p)}$ lorsqu'on relie ces grandeurs par la relation de l'équation 8, sous la forme suivante :

$$Y^0_{(n\times1)} = X^0_{(n\times p)}.B + \varepsilon \quad \text{(équation 24)}$$

Cette corrélation satisfaisante est considérée comme atteinte lorsque le résidu ε dans cette équation 24 a une valeur inférieure à une valeur de seuil $V_0$ prédéterminée en fonction de la précision recherchée pour la méthode de calcul de l'indice de basicité. Si tel est le cas, on considère que l'équation 24 est satisfaite et on arrête l'itération dans une étape β12. Dans le cas contraire, l'itération continue en revenant aux étapes β3 et β4. Un calcul de l'erreur quadratique moyenne permet également de déterminer K et d'arrêter l'itération à l'étape β12.

On explicite ci-après en détail, à titre d'exemple, les deux premiers pas d'itération des étapes β5 à β12.

Par définition des équations 6 et 7, on a :

$$X_{(n\times p)} = \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix}, \qquad Y_{(n\times 1)} = \begin{bmatrix} y_1 \\ \vdots \\ y_n \end{bmatrix}$$

1 Pas d'itération 1 : Pour $k = 1 \Rightarrow X = X^0$ et $Y = Y^0$

*1.1 Calcul de $T_1$ à l'étape $\beta 3$ et $C_1$ à l'étape $\beta 4$*

$$T_1 = X^0\, W_1 = \begin{bmatrix} x_{11}^0 & x_{12}^0 & \cdots & x_{1p}^0 \\ x_{21}^0 & x_{22}^0 & \cdots & x_{2p}^0 \\ \vdots & \vdots & & \vdots \\ x_{n1}^0 & x_{n2}^0 & \cdots & x_{np}^0 \end{bmatrix} \begin{bmatrix} w_{11} & w_{12} & \cdots & w_{1p} \\ w_{21} & w_{22} & \cdots & w_{2p} \\ \vdots & \vdots & & \vdots \\ w_{p1} & w_{p2} & \cdots & w_{pp} \end{bmatrix}$$

$$T_1 = \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix}$$

$$C_1 = Y^0 U_1 = \begin{bmatrix} y_1^0 \\ \vdots \\ y_n^0 \end{bmatrix} [U_1] = \begin{bmatrix} c_1 \\ \vdots \\ c_n \end{bmatrix}$$

*1.2 Calcul de $W_1$ et $U_1$ à l'étape $\beta 5$*

$$W_1 = {}^tX_0\, Y_0\, {}^tY_0\, X_0$$

$$W_1 = \begin{bmatrix} x_{11}^0 & x_{21}^0 & \cdots & x_{n1}^0 \\ x_{12}^0 & x_{22}^0 & \cdots & x_{n2}^0 \\ \vdots & \vdots & & \vdots \\ x_{1p}^0 & x_{2p}^0 & \cdots & x_{np}^0 \end{bmatrix} \begin{bmatrix} y_1^0 \\ \vdots \\ y_n^0 \end{bmatrix} \begin{bmatrix} y_1^0 & \cdots & y_n^0 \end{bmatrix} \begin{bmatrix} x_{11}^0 & x_{12}^0 & \cdots & x_{1p}^0 \\ x_{21}^0 & x_{22}^0 & \cdots & x_{2p}^0 \\ \vdots & \vdots & & \vdots \\ x_{n1}^0 & x_{n2}^0 & \cdots & x_{np}^0 \end{bmatrix}$$

$$W_1 = \begin{bmatrix} w_{11} & w_{12} & \cdots & w_{1p} \\ w_{21} & w_{22} & \cdots & w_{2p} \\ \vdots & \vdots & & \vdots \\ w_{p1} & w_{p2} & \cdots & w_{pp} \end{bmatrix}$$

$$U_1 = {}^tX^0 X^0\, {}^tX^0 Y^0$$

$$U_1 = [y_1^0 \quad \cdots \quad y_n^0] \begin{bmatrix} x_{11}^0 & x_{12}^0 & \cdots & x_{1p}^0 \\ x_{21}^0 & x_{22}^0 & \cdots & x_{2p}^0 \\ \vdots & \vdots & & \vdots \\ x_{n1}^0 & x_{n2}^0 & \cdots & x_{np}^0 \end{bmatrix} \begin{bmatrix} x_{11}^0 & x_{21}^0 & \cdots & x_{n1}^0 \\ x_{12}^0 & x_{22}^0 & \cdots & x_{n2}^0 \\ \vdots & \vdots & & \vdots \\ x_{1p}^0 & x_{2p}^0 & \cdots & x_{np}^0 \end{bmatrix} \begin{bmatrix} y_1^0 \\ \vdots \\ y_n^0 \end{bmatrix}$$

$$U_1 = [U_1]$$

*1.3 Calcul des coefficients de régression $p_1$ à l'étape β6 et $q_1$ à l'étape β7*

$$p_1 = \frac{{}^tX^0 T_1}{{}^tT_1 T_1} = ({}^tT_1 T_1)^{-1}\, {}^tX_0\, T_1$$

$$p_1 = IV_{1(p\times p)} \begin{bmatrix} x_{11}^0 & x_{12}^0 & \cdots & x_{1p}^0 \\ x_{21}^0 & x_{22}^0 & \cdots & x_{2p}^0 \\ \vdots & \vdots & & \vdots \\ x_{n1}^0 & x_{n2}^0 & \cdots & x_{np}^0 \end{bmatrix} \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix}$$

$$p_1 = \begin{bmatrix} p_{11} & p_{12} & \cdots & p_{1p} \\ p_{21} & p_{22} & \cdots & p_{2p} \\ \vdots & \vdots & & \vdots \\ p_{p1} & p_{p2} & \cdots & p_{pp} \end{bmatrix}$$

$$q_1 = \frac{{}^tY_0\, T_1}{{}^tT_1 T_1} = {}^tY_0\, T_1\, ({}^tT_1 T_1)^{-1} = [y_1^0 \quad \cdots \quad y_n^0] \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix} IV_{1(p\times p)}$$

$$q_1 = [q_1 \quad \cdots \quad q_p]$$

Dans ce qui précède, la notation $IV_{1(p\times p)}$ correspond au résultat du calcul de la matrice inverse de ${}^tT_1T_1$ dans la formule précédente. Il s'agit d'une matrice carrée.

*1.4 Régression de $X_1$ et $Y_1$ sur $T_1$ aux étapes β6 et β7*

$$X_1 = X^0 - T_1\, {}^tp_1$$

$$X_1 = \begin{bmatrix} x_{11}^0 & x_{12}^0 & \cdots & x_{1p}^0 \\ x_{21}^0 & x_{22}^0 & \cdots & x_{2p}^0 \\ \vdots & \vdots & & \vdots \\ x_{n1}^0 & x_{n2}^0 & \cdots & x_{np}^0 \end{bmatrix} - \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix} \begin{bmatrix} p_{11} & p_{12} & \cdots & p_{1p} \\ p_{21} & p_{22} & \cdots & p_{2p} \\ \vdots & \vdots & & \vdots \\ p_{p1} & p_{p2} & \cdots & p_{pp} \end{bmatrix}$$

$$Y_1 = Y^0 - T_1{}^t q_1$$

$$Y_1 = \begin{bmatrix} y_1^0 \\ \vdots \\ y_n^0 \end{bmatrix} - \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix} \begin{bmatrix} q_1 \\ \vdots \\ q_p \end{bmatrix}$$

2 Pas d'itération 2 : Pour $k = 2 \Rightarrow X = X_1$ *et* $Y = Y_1$

*2.1 Calcul de $T_2$ à l'étape β3 et $C_2$ à l'étape β4*

$$T_2 = X_1 W_2 = \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix} \begin{bmatrix} w_{11} & w_{12} & \cdots & w_{1p} \\ w_{21} & w_{22} & \cdots & w_{2p} \\ \vdots & \vdots & & \vdots \\ w_{p1} & w_{p2} & \cdots & w_{pp} \end{bmatrix}$$

$$T_2 = \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix}$$

$$C_2 = Y_1 U_2 = \begin{bmatrix} y_1 \\ \vdots \\ y_n \end{bmatrix} [U_1] = \begin{bmatrix} c_1 \\ \vdots \\ c_n \end{bmatrix}$$

*2.2 Calcul de $W_2$ et $U_2$ à l'étape β5*

$$W_2 = {}^tX_1\, Y_1\, {}^tY_1\, X_1$$

$$W_2 = \begin{bmatrix} x_{11} & x_{21} & \cdots & x_{n1} \\ x_{12} & x_{22} & \cdots & x_{n2} \\ \vdots & \vdots & & \vdots \\ x_{1p} & x_{n2} & \cdots & x_{np} \end{bmatrix} \begin{bmatrix} y_1 \\ \vdots \\ y_n \end{bmatrix} [y_1 \quad \cdots \quad y_n] \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix}$$

$$W_2 = \begin{bmatrix} w_{11} & w_{12} & \cdots & w_{1p} \\ w_{21} & w_{22} & \cdots & w_{2p} \\ \vdots & \vdots & & \vdots \\ w_{p1} & w_{p2} & \cdots & w_{pp} \end{bmatrix}$$

$$U_2 = {}^tY_1\, X_1\, {}^tX_1\, Y_1$$

$$U_2 = [y_1 \quad \cdots \quad y_n] \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix} \begin{bmatrix} x_{11} & x_{21} & \cdots & x_{n1} \\ x_{12} & x_{22} & \cdots & x_{n2} \\ \vdots & \vdots & & \vdots \\ x_{1p} & x_{n2} & \cdots & x_{np} \end{bmatrix} \begin{bmatrix} y_1 \\ \vdots \\ y_n \end{bmatrix}$$

$$U_2 = [U_2]$$

*2.3 Calcul des coefficients de régression $p_2$ à l'étape β6 et $q_2$ à l'étape β7*

$$p_2 = \frac{{}^tX_1\, T_2}{{}^tT_2 T_2} = ({}^tT_2 T_2)^{-1}\, {}^tX_1\, T_2$$

$$p_2 = IV_{2(p \times p)} \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix} \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix}$$

$$p_2 = \begin{bmatrix} p_{11} & p_{12} & \cdots & p_{1p} \\ p_{21} & p_{22} & \cdots & p_{2p} \\ \vdots & \vdots & & \vdots \\ p_{p1} & p_{p2} & \cdots & p_{pp} \end{bmatrix}$$

$$q_2 = \frac{{}^tY_0\, T_1}{{}^tT_1 T_1} = {}^tY_0\, T_1\, ({}^tT_1 T_1)^{-1} = [y_1 \quad \cdots \quad y_n] \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix} IV_{1(p \times p)}$$

$$q_2 = [q_1 \quad \cdots \quad q_p]$$

*2.4 Régression de $X_2$ et $Y_2$ sur $T_2$ aux étapes β6 et β7*

$$X_2 = X_1 - T_2\, {}^tp_2$$

$$X_2 = \begin{bmatrix} x_{11} & x_{12} & \cdots & x_{1p} \\ x_{21} & x_{22} & \cdots & x_{2p} \\ \vdots & \vdots & & \vdots \\ x_{n1} & x_{n2} & \cdots & x_{np} \end{bmatrix} - \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix} \begin{bmatrix} p_{11} & p_{12} & \cdots & p_{1p} \\ p_{21} & p_{22} & \cdots & p_{2p} \\ \vdots & \vdots & & \vdots \\ p_{p1} & p_{p2} & \cdots & p_{pp} \end{bmatrix}$$

$$Y_2 = Y_1 - T_2\, {}^tq_2$$

$$Y_2 = \begin{bmatrix} y_1 \\ \vdots \\ y_n \end{bmatrix} - \begin{bmatrix} t_{11} & t_{12} & \cdots & t_{1p} \\ t_{21} & t_{22} & \cdots & t_{2p} \\ \vdots & \vdots & & \vdots \\ t_{n1} & t_{n2} & \cdots & t_{np} \end{bmatrix} \begin{bmatrix} q_1 \\ \vdots \\ q_p \end{bmatrix}$$

Les pas d'itération suivants se déduisent des deux premiers, sur la base des explications qui précèdent.

Lorsque le pas d'itération K a été atteint à l'étape β12, on obtient, lors d'une étape γ1 de synthèse, l'équation générale 25 suivante, en appliquant successivement l'équation 23 pour k allant de 1 à K:

$$Y = Y^0 = T_1{}^t q_1 + T_2{}^t q_2 + \cdots + T_K{}^t q_K + Y_K = T^t q + Y_K \qquad \text{(équation 25)}$$

où la notation $T^tq$ signifie $\sum_1^K T_k{}^t q_k$

En remplaçant T par sa définition de l'équation 9, soit $T = X.\ W$, on obtient la relation :

$$Y = Y^0 = X_1 W_1{}^t q_1 + X_2 W_2{}^t q_2 + \cdots + X_K W_K{}^t q_K + Y_K$$
$$= X W^t q + Y_K \qquad \text{(équation 26)}$$

où la notation $W^tq$ signifie $\sum_1^K W_k{}^t q_k$

Par comparaison entre les équations 8 et 26, on obtient, dans une deuxième étape γ2 de synthèse, la matrice B et le résidu ε correspondant, avec les relations :

$$B = W^t q = \sum_1^K W_k{}^t q_k \qquad \text{(équation 27)}$$

$$\varepsilon = Y_K \qquad \text{(équation 28)}$$

Ainsi, la méthode des étapes α1 à α7 permet de déterminer une matrice B et un résidu ε qui peuvent être utilisés comme la matrice $M_{(px1)}$ et le résidu R du modèle dans l'équation 3, lors de l'étape **c.** En d'autres termes, les valeurs de pondération $m_i$ et le résidu R du modèle sont déterminés lors des étapes α1 à α7.

Les étapes β1 à β12, γ1 et γ2 sont en fait des sous-étapes de l'étape α7.

Mise à part, éventuellement, l'introduction des échantillons dans le réservoir 4, le calcul du modèle peut être réalisé de façon automatique par l'unité électronique 10. En d'autres termes, les étapes α1 à α7 et les sous-étapes de l'étape α7 peuvent être automatisées, comme les étapes **a** à **c.**

L'invention est décrite ci-dessus dans le cas de son utilisation pour un moteur de propulsion de navire. Elle est toutefois applicable à d'autres équipements, par exemple un moteur auxiliaire ou d'accessoires de navire, ainsi qu'une boîte à vitesses, notamment une boîte à vitesses d'hydrolienne ou d'éolienne, ou un moteur à combustion interne de centrale électrique.

Dans le deuxième mode de réalisation représenté aux figures 5 et 6, les étapes analogues à celles du premier mode de réalisation portent les mêmes références.

Dans ce qui suit, on décrit uniquement ce qui distingue ce deuxième mode de réalisation du précédent.

Dans ce deuxième mode de réalisation, l'étape b) de calcul de la valeur de transmittance $T_r$ comprend deux étapes élémentaires successives. Une première étape élémentaire b1) consiste à calculer, pour chaque nombre d'onde N, le rapport T'r (N) de l'intensité I(N) du signal infra rouge F passant à travers l'échantillon E sur l'intensité à vide $I_0$(N). Cette étape élémentaire b1) est comparable à l'étape b) du premier mode de réalisation mais ne conduit pas directement à la détermination de la transmittance.

En effet, l'étape b) de ce deuxième mode de réalisation comprend une deuxième étape élémentaire de calcul effectif de la transmittance Tr(N) comme une valeur corrigée du rapport T'r(N). La correction consiste à exprimer la valeur de la transmittance Tr(N) pour un nombre d'onde N à l'aide d'une fonction f dont la variable est le rapport Tr'(N), sous la forme

$$TN(N) = f(T'r(N)) \qquad \text{(équation 29)}$$

La correction effectuée lors de l'étape b2), par application de la fonction f, peut consister à recaler la valeur du rapport T'r(N) par le retrait d'une ligne de base, ce qui revient à décaler la valeur du rapport T'r(N) d'une valeur constante ou variable en fonction des nombres d'ondes. En variante ou en complément, la correction effectuée lors de l'étape élémentaire b2) consiste à filtrer la valeur du rapport T'r(N), en d'autres termes en un lissage.

Dans ce mode de réalisation et comme visible à la figure 6, l'étape préalable $\alpha$3 est également décomposée en deux étapes élémentaires, à savoir une première étape élémentaire $\alpha$31) comparable à l'étape $\alpha$31) du premier mode de réalisation et dans laquelle on calcule un rapport T'r(N) comparable à celui mentionné ci-dessus, et une deuxième étape élémentaire $\alpha$32) dans laquelle la valeur de la transmittance Tr(N) est calculée sous la forme d'une valeur corrigée de ce rapport. La correction consiste à exprimer la valeur de la transmittance Tr(N) pour un nombre d'onde N à l'aide d'une fonction g dont la variable est le rapport T'r(N), sous la forme

$$Tr(N) = g(T'r(N)) \qquad \text{(équation 30)}$$

La fonction g est de préférence identique à la fonction f, même si ceci n'est pas obligatoire.

La correction de l'étape élémentaire $\alpha$32) peut prendre la forme d'un filtrage et/ou du retrait d'une ligne de base, comme envisagé ci-dessus pour l'étape b2).

Les étapes b2) et $\alpha$32) peuvent être considérées des étapes de prétraitement des données brutes T'r(N) pour fournir les données traitées Tr(N).

## Revendications

**1.** Procédé de détermination en ligne d'un indice de basicité (BN) d'un corps liquide (L), à caractériser, notamment un lubrifiant, par spectroscopie moyenne infra rouge, dans lequel on met en œuvre au moins une étape consistant à :

a) détecter (S14) l'intensité d'un signal infra rouge (F) passant à travers un échantillon (E) du corps liquide à caractériser,

**caractérisé en ce que** ce procédé comprend également au moins des étapes supplémentaires consistant à

b) postérieurement à l'étape **a,** calculer la valeur de transmittance ($T_r$) d'ondes infra rouge à travers l'échantillon (E) pour p nombres d'onde, avec p un entier naturel supérieur ou égal à deux ;
c) exprimer l'indice de basicité du corps liquide à caractériser sous la forme

$$BN = Tr.M_{(px1)}+R$$

où

BN est l'indice de basicité du lubrifiant à caractériser,
Tr est un jeu de valeurs de transmittance calculées à l'étape **b**
$M_{(px1)}$ est un ensemble de données de modèle contenant des coefficients déterminés à partir de valeurs d'indice de basicité mesurées et de valeurs de transmittance mesurées pour des corps liquides de référence,
R est un résidu de modèle, déterminé à partir des valeurs mesurées d'indice de basicité et de transmittance.

**2.** Procédé selon la revendication 1, **caractérisé en ce que**, lors de l'étape b), la valeur de transmittance (Tr(N)) pour chaque nombre d'onde (N) est calculée comme le rapport de l'intensité (I(N)) du signal infra rouge (F) passant à travers l'échantillon (E) sur l'intensité à vide ($I_0$(N)) du signal.

**3.** Procédé selon la revendication 1, **caractérisé en ce que** l'étape b) comprend des étapes élémentaires consistant à :

b1) calculer, pour chaque nombre d'onde (N), le rapport ($T'_r$(N)) de l'intensité (I(N)) du signal infra rouge (F) passant à travers l'échantillon (E) sur l'intensité à vide ($I_0$(N)) du signal,
b2) calculer la valeur de transmittance (Tr(N)) sous la forme d'une valeur corrigée du rapport calculé à l'étape b1).

**4.** Procédé selon la revendication 3, **caractérisé en ce que**, lors de l'étape élémentaire b2), la valeur du rapport est corrigée par filtrage et/ou retrait d'une ligne de base.

**5.** Procédé selon l'une des revendications précédentes, **caractérisé en ce que** la valeur de transmittance comprend une matrice unidirectionnelle ($T_r$) à une ligne et p colonnes et l'ensemble de données du modèle est une matrice unidirectionnelle ($M_{(px1)}$) à p lignes et une colonne.

**6.** Procédé selon l'une des revendications précédentes, **caractérisé en ce qu'**il comprend des étapes préalables, mises en œuvre pour la détermination du modèle et consistant à :

$\alpha$1) détecter l'intensité ($I_0$(N)) d'un signal infra rouge (F), pour les p nombres d'onde, dans le vide,
$\alpha$2) détecter l'intensité (I(N)) d'un signal infra rouge, pour les p nombres d'onde, au sein de n échantillons, chacun représentatif d'un corps liquide de référence ;
$\alpha$3) calculer la transmittance (Tr) de chaque corps liquide de référence comme un ensemble de valeurs définies, pour chaque nombre d'onde, comme le rapport de l'intensité détectée à l'étape $\alpha$2 sur l'intensité détectée à l'étape $\alpha$1;
$\alpha$4) créer un premier ensemble ($X^0$) de données de références comprenant les valeurs de transmittance calculées à l'étape $\alpha$3 pour les n échantillons et pour les p nombres d'onde ;
$\alpha$5) mesurer l'indice de basicité de chaque corps liquide de référence ;
$\alpha$6) créer un deuxième ensemble ($Y^0$) de données de référence comprenant les n valeurs d'indice de basicité mesurées à l'étape $\alpha$5 ; et
$\alpha$7) déterminer par calcul l'ensemble de données ($M_{(px1)}$) et le résidu (R) du modèle, sur la base des premier et deuxième ensembles ($X^0$, $Y^0$) de données de référence.

**7.** Procédé selon la revendication 6, **caractérisé en ce que** lors de l'étape $\alpha$3, la transmittance de chaque corps liquide de référence est calculée comme un ensemble de valeurs définies, pour chaque nombre d'onde, comme le rapport de l'intensité (I(N)) détectée à l'étape $\alpha$2 sur l'intensité ($I_0$(N)) détectée à l'étape $\alpha$1;

**8.** Procédé selon la revendication 6, **caractérisé en ce que** l'étape $\alpha$3 comprend des étapes élémentaires consistant à :

$\alpha$31) calculer, pour chaque nombre d'ondes (N), le rapport ($T'_r$(N)) de l'intensité (I(N)) détectée à l'étape $\alpha$2) sur l'intensité ($I_0$(N)) détectée à l'étape $\alpha$1),
$\alpha$32) calculer la valeur de transmittance (Tr(N)) sous la forme d'une valeur corrigée du rapport calculé à l'étape $\alpha$31), de préférence par filtrage et/ou retrait d'une ligne de base.

**9.** Procédé selon les revendications 5 et 6, **caractérisé en ce que** :

- le premier ensemble ($X^0$) de données de référence est une matrice bi-dimensionnelle à n lignes et p colonnes, chaque ligne comprenant des valeurs de transmittance calculées à l'étape $\alpha$3 pour un corps liquide de référence et p nombres d'onde (N) et chaque colonne comprenant des valeurs de transmittance calculées pour un nombre d'onde et les n corps liquide de référence,
- le deuxième ensemble ($Y^0$) de données de référence est une matrice unidirectionnelle à n lignes et une colonne.

**10.** Procédé selon l'une des revendications 6 à 9, **caractérisé en ce qu'**il comprend des étapes préalables supplémentaires, mises en œuvre au cours de l'étape $\alpha$7 pour la détermination du modèle et consistant à :

$\beta$1) définir un premier ensemble (X) de données de calcul qui est une image du premier ensemble ($X^0$) de données de référence ;
$\beta$2) définir un deuxième ensemble (Y) de données de calcul qui est une image du deuxième ensemble ($Y^0$) de données de référence ;
$\beta$3) définir une première variable (T) dépendante du premier ensemble de données de calcul selon la relation : $T = X.W$

où T est la première variable,
X est le premier ensemble de données de calcul,
W est un premier coefficient de pondération,

$\beta$4) définir une deuxième variable (C) dépendante du deuxième ensemble de données de calcul selon la relation :

C = Y.U

où C est la deuxième variable,
Y est le deuxième ensemble de données de calcul,
U est un deuxième coefficient de pondération, et

β5) calculer de façon itérative, les valeurs des deux coefficients de pondération (W, U), en maximisant la covariance des premier et deuxième variables (T, C).

**11.** Procédé selon la revendication 10, **caractérisé en ce qu'**il comprend des étapes préalables supplémentaires mises en œuvre au cours de l'étape α7 pour la détermination du modèle et consistant à :

β6) appliquer, à chaque pas itératif (k), une régression linéaire du premier ensemble (X) de données de calcul sur la première variable (T) ;
β7) appliquer, à chaque pas itératif (k), une régression linéaire du deuxième ensemble (Y) de données de calcul sur la première variable ;
β8) calculer, à chaque pas itératif (k), une valeur estimée (X'k) du premier ensemble de données de calcul, en appliquant une transposée ($^t p_k$) d'une pente ($p_k$) de la régression linéaire, déterminée à ce pas, à la première variable (Tk) calculée à ce pas.
β9) calculer, à chaque pas itératif (k), une valeur réelle ($X_k$) du premier ensemble (X) de données de calcul, en soustrayant la valeur estimée (X'k) calculée à l'étape β8 d'une valeur réelle ($X_{k-1}$) de ce premier ensemble de données de calcul calculée au pas itératif précédent (k-1).
β10) calculer, à chaque pas itératif (k) de l'étape β5), une valeur estimée (Y'k) du deuxième ensemble (Y) de données de calcul, en appliquant une transposée ($^t p_k$) d'une pente ($q_k$) de la régression linéaire, déterminée à ce pas, à la première variable (Tk) calculée à ce pas.
β11) calculer, à chaque pas itératif (k), une valeur réelle ($Y_k$) du deuxième ensemble (Y) de données de calcul, en soustrayant la valeur estimée (Y'k) calculée à l'étape β10 d'une valeur réelle ($Y_{k-1}$) de ce deuxième ensemble de données de calcul calculée au pas itératif précédent (k-1).

**12.** Procédé selon la revendication 11, **caractérisé en ce que** les étapes β5 à β11 sont répétées jusqu'à ce que la valeur du deuxième ensemble de données devienne inférieure à une valeur de seuil prédéterminée ($V_0$)

**13.** Procédé selon la revendication 9 et l'une des revendications 10 à 12, **caractérisé en ce que**

- la première variable (T) est une matrice bi-dimensionnelle à n lignes et p colonnes,
- la deuxième variable (C) est une matrice unidirectionnelle à n lignes et une colonne,
- le premier coefficient de pondération (W) est une matrice bi-dimensionnelle à p lignes et p colonnes,
- le deuxième coefficient de pondération (U) est un nombre,
- à chaque pas itératif (k), les coefficients de pondération ($W_k$, $U_k$) sont calculés selon les équations :

$$W_k = {}^tX_{k-1}\,Y_{k-1}\,{}^tY_{k-1}\,X_{k-1}$$

$$U_k = {}^tY_{k-1}\,X_{k-1}\,{}^tX_{k-1}\,Y_{k-1}$$

où l'indice k appliqué à une matrice indique la valeur de cette matrice au pas d'itération k
- la régression linéaire de l'étape β6 est effectuée sur la base d'une équation de type :

$$X_{k-1} = T_k\,{}^tp_k + X_{k_k}$$

où $p_k$ est une matrice de coefficients de régression déterminée par la méthode des moindres carrés et qui s'exprime sous la forme :

$$p_k = \frac{{}^tX_{k-1}\,T_k}{{}^tT_k T_k}$$

- la régression linéaire de l'étape β7 est effectuée sur la base d'une équation de type :

$$Y_{k-1} = T_k{}^t q_k + Y_{k_k}$$

où $q_k$ est une matrice de coefficients de régression déterminée par la méthode des moindres carrés et qui s'exprime sous la forme :

$$q_k = \frac{{}^tY_{k-1}\,T_k}{{}^tT_kT_k}$$

**14.** Procédé selon l'une des revendications 11 à 13, **caractérisé en ce qu'**il comprend des étapes préalables supplémentaires, mises en œuvre pour la détermination du modèle et consistant à :

γ1) exprimer le deuxième ensemble ($Y_0$) de données de référence en fonction de la première variable selon la relation :

$$Y^0 = \sum_1^K W_k{}^t q_k + Y_K$$

où K est le nombre d'itérations de l'étape β5), k est un entier naturel entre 1 et K, Tk est la première variable au rang d'itération k,
${}^tq_k$ est la transposée de la pente de régression linéaire utilisée à l'étape β10
$Y_K$ est le deuxième ensemble de données de calcul au rang d'itération K,

γ2) déterminer l'ensemble de données du modèle ($M_{(px1)}$) et le résidu (R) comme étant respectivement égaux à

$$M_{(px1)} = \sum_1^K W_k{}^t q_k$$

$R = Y_K$
où $W_k$ est le coefficient de pondération au rang d'itération k.

**15.** Utilisation d'un procédé selon l'une des revendications précédentes pour la détermination de l'indice de basicité (BN) d'un lubrifiant circulant dans un équipement d'un navire, en particulier dans un moteur navire.

## Patentansprüche

**1.** Verfahren zur Online-Bestimmung einer Basenzahl (BN) eines zu charakterisierenden flüssigen Körpers (L), insbesondere ein Schmierstoff, durch Mittelinfrarot-Spektroskopie, bei dem wenigstens ein Schritt durchgeführt wird, bestehend aus:

a) Erfassen (S14) der Intensität eines Infrarotsignals (F), das eine Probe (E) des zu charakterisierenden flüssigen Körpers durchquert,

**dadurch gekennzeichnet, dass** dieses Verfahren ferner mindestens weitere Schritte umfasst, bestehend aus:

b) nach Schritt a), Berechnen des Infrarotwellen-Transmissionsgrades ($T_r$) durch die Probe (E) hindurch für p Wellenzahlen, wobei p eine natürliche ganze Zahl größer oder gleich zwei ist;
c) Ausdrücken der Basenzahl des zu charakterisierenden flüssigen Körpers in der Form

$$BN = Tr.M_{(px1)} + R$$

worin

BN die Basenzahl des zu charakterisierenden Schmierstoffs ist,
Tr ein Satz von Transmissionsgraden ist, die in Schritt b berechnet wurden,
$M_{(px1)}$ ein Satz von Modelldaten ist, die Koeffizienten enthalten, die ausgehend von für flüssige Referenzkörper gemessenen Basenzahlwerten und Transmissionsgraden bestimmt werden,
R ein Modellrückstand ist, der ausgehend von den gemessenen Basenzahl- und Transmissionsgraden bestimmt wird.

**2.** Verfahren nach Anspruch 1, **dadurch gekennzeichnet, dass** bei Schritt b) der Transmissionsgrad (Tr(N)) für jede Wellenzahl (N) berechnet wird als das Verhältnis der Intensität (I(N)) des Infrarotsignals (F), das die Probe (E) durchquert, zu der Intensität ($I_0$(N)) des Signals im leeren Raum.

**3.** Verfahren nach Anspruch 1, **dadurch gekennzeichnet, dass** Schritt b) wesentliche Schritte umfasst, bestehend aus:

b1) Berechnen, für jede Wellenzahl (N), des Verhältnisses ($T'_r$(N)) der Intensität (I(N)) des Infrarotsignals (F), das die Probe (E) durchquert, zu der Intensität im leeren Raum ($I_0$(N)) des Signals,
b2) Berechnen des Transmissionsgrades (Tr(N)) in Form eines Wertes, der um das in Schritt b1) berechnete Verhältnis korrigiert ist.

**4.** Verfahren nach Anspruch 3, **dadurch gekennzeichnet, dass** beim wesentlichen Schritt b2) der Wert des Verhältnisses durch Filtern und/oder Entfernen einer Grundlinie korrigiert wird.

**5.** Verfahren nach einem der vorhergehenden Ansprüche, **dadurch gekennzeichnet, dass** der Transmissionsgrad eine unidirektionale Matrix ($T_r$) mit einer Zeile und p Spalten umfasst und der Satz von Modelldaten eine unidirektionale Matrix ($M_{(px1)}$) mit p Zeilen und einer Spalte ist.

**6.** Verfahren nach einem der vorhergehenden Ansprüche, **dadurch gekennzeichnet, dass** es vorherige Schritte umfasst, die zur Bestimmung des Modells durchgeführt werden, und bestehend aus:

$\alpha$1) Erfassen der Intensität ($I_0$(N)) eines Infrarotsignals (F), für die p Wellenzahlen, im leeren Raum,
$\alpha$2) Erfassen der Intensität (I(N)) eines Infrarotsignals, für die p Wellenzahlen, in n Proben, die jede einen flüssigen Referenzkörper darstellt;
$\alpha$3) Berechnen des Transmissionsgrades (Tr) jedes flüssigen Referenzkörpers als ein Satz definierter Werte, für jede Wellenzahl, als das Verhältnis der in Schritt $\alpha$2) erfassten Intensität zu der in Schritt $\alpha$1) erfassten Intensität;
$\alpha$4) Erzeugen eines ersten Satzes ($X^0$) von Referenzdaten, welche die Transmissionsgrade enthalten, die in Schritt $\alpha$3) für die n Proben und für die p Wellenzahlen berechnet wurden;
$\alpha$5) Messen der Basenzahl jedes flüssigen Referenzkörpers;
$\alpha$6) Erzeugen eines zweiten Satzes ($Y^0$) von Referenzdaten, welche die n Basenzahlwerte enthalten, die in Schritt $\alpha$5) gemessen wurden; und
$\alpha$7) Bestimmen, durch Berechnung, des Satzes an Daten ($M_{(px1)}$) und des _Modellrückstands (R), auf der Grundlage des ersten und zweiten Satzes ($X^0$, $Y^0$) von Referenzdaten.

**7.** Verfahren nach Anspruch 6, **dadurch gekennzeichnet, dass** bei Schritt $\alpha$3) der Transmissionsgrad jedes flüssigen Referenzkörpers als ein Satz definierter Werte, für jede Wellenzahl, berechnet wird, als das Verhältnis der in Schritt $\alpha$2) erfassten Intensität (I(N)) zu der in Schritt $\alpha$1) erfassten Intensität ($I_0$(N)).

**8.** Verfahren nach Anspruch 6, **dadurch gekennzeichnet, dass** der Schritt $\alpha$3) wesentliche Schritte umfasst, bestehend aus:

$\alpha$31) Berechnen, für jede Wellenzahl (N), des Verhältnisses ($T'_r$(N)) der in Schritt $\alpha$2) erfassten Intensität (I(N)) zu der in Schritt $\alpha$1) erfassten Intensität ($I_0$(N)),
$\alpha$32) Berechnen des Transmissionsgrades (Tr(N)) in Form eines Wertes, der um das in Schritt $\alpha$31) berechnete Verhältnis korrigiert ist, bevorzugt durch Filtern und/oder Entfernen einer Grundlinie.

**9.** Verfahren nach Anspruch 5 und 6, **dadurch gekennzeichnet, dass**:

- der erste Satz ($X^0$) von Referenzdaten eine zweidimensionale Matrix mit n Zeilen und p Spalten ist, wobei jede Zeile Transmissionsgrade enthält, die in Schritt α3) für einen flüssigen Referenzkörper und p Wellenzahlen (N) berechnet wurden, und wobei jede Spalte Transmissionsgrade enthält, die für eine Wellenzahl und die n flüssigen Referenzkörper berechnet wurden,
- der zweite Satz ($Y^0$) von Referenzdaten eine unidirektionale Matrix mit n Zeilen und einer Spalte ist.

**10.** Verfahren nach einem der Ansprüche 6 bis 9, **dadurch gekennzeichnet, dass** es weitere vorherige Schritte umfasst, die im Laufe des Schrittes α7 zur Bestimmung des Modells durchgeführt werden und bestehend aus:

β1) Definieren eines ersten Satzes (X) von Rechendaten, der eine Abbildung des ersten Satzes ($X^0$) von Referenzdaten ist;
β2) Definieren eines zweiten Satzes (Y) von Rechendaten, der eine Abbildung des zweiten Satzes ($Y^0$) von Referenzdaten ist;
β3) Definieren einer ersten Variable (T), die von dem ersten Satz von Rechendaten abhängig ist, gemäß der Relation:

$$T = X.W$$

worin

T die erste Variable ist,
X der erste Satz von Rechendaten ist,
W ein erster Gewichtungskoeffizient ist,

β4) Definieren einer zweiten Variable (C), die von dem zweiten Satz von Rechendaten abhängig ist, gemäß der Relation: C = Y.U
worin

C die zweite Variable ist,
Y der zweite Satz von Rechendaten ist,
U ein zweiter Gewichtungskoeffizient ist, und

β5) iteratives Berechnen der Werte der zwei Gewichtungskoeffizienten (W, U), indem die Kovarianz der ersten und zweiten Variable (T, C) maximiert wird.

**11.** Verfahren nach Anspruch 10, **dadurch gekennzeichnet, dass** es weitere vorherige Schritte umfasst, die im Laufe des Schrittes α7 zur Bestimmung des Modells durchgeführt werden und bestehend aus:

β6) Anwenden, in jedem iterativen Schritt (k), einer linearen Regression des ersten Satzes (X) von Rechendaten an die erste Variable (T);
β7) Anwenden, in jedem iterativen Schritt (k), einer linearen Regression des zweiten Satzes (Y) von Rechendaten an die erste Variable;
β8) Berechnen, in jedem iterativen Schritt (k), eines geschätzten Wertes (X'k) des ersten Satzes von Rechendaten, durch Anwendung einer transponierten Matrix (${}^t p_k$) einer Steigung ($p_k$) der linearen Regression, die in diesem Schritt bestimmt wird, an die erste Variable (Tk), die in diesem Schritt berechnet wird;
β9) Berechnen, in jedem iterativen Schritt (k), eines tatsächlichen Wertes ($X_k$) des ersten Satzes (X) von Rechendaten, durch Subtrahieren des geschätzten Wertes (X'k), der in Schritt β8 berechnet wurde, von einem tatsächlichen Wert ($X_{k-1}$) dieses ersten Satzes von Rechendaten, der in dem vorherigen iterativen Schritt (k-1) berechnet wurde;
β10) Berechnen, in jedem iterativen Schritt (k) des Schrittes β5), eines geschätzten Wertes (Y'k) des zweiten Satzes (Y) von Rechendaten, durch Anwendung einer transponierten Matrix (${}^t p_k$) einer Steigung ($q_k$) der linearen Regression, die in diesem Schritt bestimmt wird, an die erste Variable (Tk), die in diesem Schritt berechnet wird;
β11) Berechnen, in jedem iterativen Schritt (k), eines tatsächlichen Wertes ($Y_k$) des zweiten Satzes (Y) von Rechendaten, durch Subtrahieren des geschätzten Wertes (Y'k), der in Schritt β10 berechnet wurde, von einem tatsächlichen Wertes ($Y_{k-1}$) dieses zweiten Satzes von Rechendaten, der in dem vorherigen iterativen Schritt (k-1) berechnet wurde.

**12.** Verfahren nach Anspruch 11, **dadurch gekennzeichnet, dass** die Schritte β5 bis β11 wiederholt werden, bis der Wert des zweiten Satzes von Daten kleiner wird als ein vorher bestimmter Schwellenwert ($V_0$).

**13.** Verfahren nach Anspruch 9, und einem der Ansprüche 10 bis 12, **dadurch gekennzeichnet, dass**

- die erste Variable (T) eine zweidimensionale Matrix mit n Zeilen und p Spalten ist,
- die zweite Variable (C) eine unidirektionale Matrix mit n Zeilen und einer Spalte ist,
- der erste Gewichtungskoeffizient (W) eine zweidimensionale Matrix mit p Zeilen und p Spalten ist,
- der zweite Gewichtungskoeffizient (U) eine Zahl ist,
- bei jedem iterativen Schritt (k) die Gewichtungskoeffizienten ($W_k$, $U_k$) gemäß den folgenden Gleichungen berechnet werden:

$$W_k = {}^tX_{k-1}Y_{k-1}{}^tY_{k-1}X_{k-1}$$

$$U_k = {}^tY_{k-1}X_{k-1}{}^tX_{k-1}Y_{k-1}$$

worin der auf eine Matrix angewendete Index k den Wert dieser Matrix im iterativen Schritt k angibt,
- die lineare Regression aus dem Schritt β6 durchgeführt wird auf der Grundlage einer Gleichung des Typs:

$$X_{k-1} = T_k{}^tp_k + X_{k_k}$$

worin
$p_k$ eine Regressionskoeffizientenmatrix ist, die durch die Methode der kleinsten Quadrate bestimmt wird und die in folgender Form ausgedrückt wird:

$$p_k = \frac{{}^tX_{k-1}T_k}{{}^tT_kT_k}$$

- die lineare Regression aus dem Schritt β7 durchgeführt wird auf der Grundlage einer Gleichung des Typs:

$$Y_{k-1} = T_k{}^tq_k + Y_{k_k}$$

worin
$q_k$ eine Regressionskoeffizientenmatrix ist, die durch die Methode der kleinsten Quadrate bestimmt wird und die in folgender Form ausgedrückt wird:

$$q_k = \frac{{}^tY_{k-1}T_k}{{}^tT_kT_k}$$

**14.** Verfahren nach einem der Ansprüche 11 bis 13, **dadurch gekennzeichnet, dass** es weitere vorherige Schritte umfasst, die zur Bestimmung des Modells durchgeführt werden und bestehend aus:

γ1) Ausdrücken des zweiten Satzes ($Y_0$) von Referenzdaten in Abhängigkeit der ersten Variable gemäß der Relation:

$$Y^0 = \sum_{1}^{K} W_k{}^tq_k + Y_K$$

worin

K die Anzahl der Iterationen von Schritt β5 ist, k eine natürliche ganze Zahl zwischen 1 und K ist, Tk die erste Variable im Iterationsrang k ist,
${}^{t}q_k$ die transponierte Matrix der Steigung der linearen Regression ist, die in dem Schritt β10 verwendet wird,
$Y_k$ der zweite Satz von Rechendaten im Iterationsrang K ist,

γ2) Bestimmen des Satzes von Modelldaten ($M_{(px1)}$) und des Rückstands (R) als entsprechend gleichwertig zu

$$M_{(px1)} = \sum_1^K W_k {}^{t}q_k$$

$R = Y_k$
worin
$W_k$ der Gewichtungskoeffizient im Iterationsrang k ist.

**15.** Verwendung eines Verfahrens nach einem der vorhergehenden Ansprüche zur Bestimmung der Basenzahl (BN) eines Schmierstoffs, der in einer Einrichtung eines Wasserfahrzeugs zirkuliert, insbesondere eines Motorwasserfahrzeugs.

## Claims

**1.** A method for the on-line determination of a basicity index (BN) of a liquid body (L) to be **characterized, in** particular a lubricant, by mid-infrared spectroscopy, in which at least one step is carried out consisting of:

a) detecting (S14) the intensity of an infrared signal (F) passing through a sample (E) of the liquid body to be characterized,

**characterized in that** this method also comprises at least the additional steps consisting of:

b) subsequent to step **a**, calculating the transmittance value ($T_r$) of infrared waves through the sample (E) for p wave numbers, where p is a natural integer greater than or equal to two;
c) expressing the basicity index of the liquid body to be **characterized in** the form

$$BN = Tr.M_{(px1)}+R$$

where

BN is the basicity index of the lubricant to be characterized,
Tr is a set of transmittance values calculated in step **b**
$M_{(px1)}$ is a set of model data containing the coefficients determined from the measured values of the basicity index and from the measured transmittance values for the reference liquid bodies,
R is a residue of the model, determined from the measured basicity and transmittance values.

**2.** The method according to claim 1, **characterized in that** during step b), the transmittance value (Tr(N)) for each wave number (N) is calculated as the ratio of the intensity (I(N)) of the infrared signal (F) passing through the sample (E) to the intensity ($I_0$(N)) of the signal, when empty.

**3.** The method according to claim 1, **characterized in that** step b) comprises elementary steps consisting of:

b1) calculating, for each wave number (N), the ratio (T'r(N)) of the intensity (I(N)) of the infrared signal (F) passing through the sample (E) to the intensity ($I_0$(N)) of the signal, when empty
b2) calculating the transmittance value (Tr(N)) in the form of a corrected value of the ratio calculated in step b1).

**4.** The method according to claim 3, **characterized in that** during elementary step b2), the value of the ratio is corrected by filtering and/or removal of a baseline.

**5.** The method according to one of the preceding claims, **characterized in that** the transmittance value comprises a unidirectional matrix ($T_r$) with one row and p columns and the set of model data is a unidirectional matrix ($M_{(px1)}$) with p rows and one column.

**6.** The method according to one of the preceding claims, **characterized in that** it comprises prior steps, carried out to determine the model and consisting of:

$\alpha$1) detecting the intensity ($I_0$(N)) of an infrared signal (F), for the p wave numbers, when empty,
$\alpha$2) detecting the intensity (I(N)) of an infrared signal, for the p wave numbers, within n samples, each representative of a reference liquid body;
$\alpha$3) calculating the transmittance (Tr) of each reference liquid body as a set of values defined, for each wave number, as the ratio of the intensity detected in step $\alpha$2 to the intensity detected in step $\alpha$1;
$\alpha$4) creating a first set ($X^0$) of reference data comprising the transmittance values calculated in step $\alpha$3 for the n samples and for the p wave numbers;
$\alpha$5) measuring the basicity index of each reference liquid body;
$\alpha$6) creating a second set of reference data ($Y^0$) comprising the n basicity index values measured in step $\alpha$5; and
$\alpha$7) determining, by calculation, the set of data ($M_{(px1)}$) and the residue (R) of the model, based on the first and second sets ($X^0$, $Y^0$) of reference data.

**7.** The method according to claim 6, **characterized in that** during step $\alpha$3), the transmittance of each reference liquid body is calculated as a set of values defined, for each wave number, as the ratio of the intensity (I(N)) detected in step $\alpha$2) to the intensity ($I_0$(N)) detected in step $\alpha$1).

**8.** The method according to claim 6, **characterized in that** step $\alpha$3 comprises elementary steps consisting of:

$\alpha$31) calculating, for each wave number (N), the ratio ($T'_r$(N)) of the intensity (I(N)) detected in step $\alpha$2) to the intensity ($I_0$(N)) detected in step $\alpha$1),
$\alpha$32) calculating the transmittance value (Tr(N)) in the form of a corrected value of the ratio calculated in step $\alpha$31), preferably by filtering and/or removing a baseline.

**9.** The method according to claims 5 and 6, **characterized in that**:

- the first set ($X^0$) of reference data is a two-dimensional matrix with n rows and p columns, each row comprising transmittance values calculated in step $\alpha$3 for a reference liquid body and p wave numbers and each column comprising transmittance values calculated for a wave number and the n reference liquid bodies,
- the second set ($Y^0$) of reference data is a unidirectional matrix with n rows and one column.

**10.** The method according to one of claims 6 to 9, **characterized in that** it comprises additional prior steps, carried out during step $\alpha$7 to determine the model and consisting of:

$\beta$1) defining a first set (X) of calculation data that is an image of the first set ($X^0$) of reference data;
$\beta$2) defining a second set (Y) of calculation data that is an image of the second set ($Y^0$) of reference data;
$\beta$3) defining a first variable (T) dependent on the first set of calculation data according to the relationship: $T = X.W$

where T is the first variable,
X is the first set of calculation data,
W is a first weight coefficient,

$\beta$4) defining a second variable (C) dependent on the second set of calculation data according to the relationship: $C = Y.U$

where C is the second variable,
Y is the second set of calculation data,
U is a second weight coefficient, and

$\beta$5) calculating the values of the two weight coefficients (W, U) iteratively, while maximizing the covariance of the first and second variables (T, C).

**11.** The method according to claim 10, **characterized in that** it comprises additional prior steps, carried out during step $\alpha 7$ to determine the model and consisting of:

$\beta 6$) applying, to each iteration (k), a linear regression of the first set (X) of calculation data on the first variable (T);
$\beta 7$) applying, to each iteration (k), a linear regression of the second set (Y) of calculation data on the first variable;
$\beta 8$) calculating, in each iteration (k), an estimated value (X'k) of the first set of calculation data, by applying a transpose (${}^t p_k$) of a slope ($p_k$) of the linear regression, determined **in that** step, to the first calculated variable (Tk) **in that** step.
$\beta 9$) calculating, in each iteration (k), an actual value ($X_k$) of the first set (X) of calculation data, by subtracting the estimated value (X'k) calculated in step $\beta 8$ from an actual value ($X_{k-1}$) of this first set of calculation data calculated in the previous iteration (k-1).
$\beta 10$) calculating, in each iteration (k) of step $\beta 5$, an estimated value (Y') of the second set (Y) of calculation data, by applying a transpose (${}^t p_k$) of a slope ($q_k$) of the linear regression, determined **in that** iteration, to the first calculated variable (Tk) **in that** iteration.
$\beta 11$) calculating, in each iteration (k), an actual value ($Y_k$) of the second set (Y) of calculation data, by subtracting the estimated value (Y'k) calculated in step $\beta 10$ from an actual value ($Y_{k-1}$) of this second set of calculation data calculated in the previous iteration (k-1).

**12.** The method according to claim 11, **characterized in that** steps $\beta 5$ to $\beta 11$ are repeated until the value of the second set of data becomes lower than a predetermined threshold value ($V_0$).

**13.** The method according to claim 9 and one of claims 10 to 12, **characterized in that**

- the first variable (T) is a two-dimensional matrix with n rows and p columns,
- the second variable (C) is a unidirectional matrix with n rows and one column,
- the first weight coefficient (W) is a two-dimensional matrix with p rows and p columns,
- the second weight coefficient (U) is a number,
- in each iteration (k), the weight coefficients ($W_k$, $U_k$) are calculated according to the equations:

$$W_k = {}^tX_{k-1} Y_{k-1} {}^tY_{k-1} X_{k-1}$$

$$U_k = {}^tY_{k-1} X_{k-1} {}^tX_{k-1} Y_{k-1}$$

where the index k applied to a matrix indicates the value of that matrix in iteration k,
- the linear regression of step $\beta 6$ is done based on an equation of the type:

$$X_{k-1} = T_k {}^tp_k + X_{k_k}$$

where $p_k$ is a matrix of regression coefficients determined using the method of least squares and expressed in the form:

$$p_k = \frac{{}^tX_{k-1} T_k}{{}^tT_k T_k}$$

- the linear regression of step $\beta 7$ is done based on an equation of the type:

$$Y_{k-1} = T_k {}^tq_k + Y_{k_k}$$

where $q_k$ is a matrix of regression coefficients determined by the method of least squares and that is expressed in the form:

$$q_k = \frac{{}^tY_{k-1}\,T_k}{{}^tT_kT_k}$$

**14.** The method according to one of claims 11 to 13, **characterized in that** it comprises additional prior steps, carried out to determine the model and consisting of:

γ1) expressing the second set ($Y_0$) of reference data as a function of the first variable according to the relationship:

$$Y^0 = \sum_{1}^{K} W_k\ {}^tq_k + Y_K$$

where K is the number of iterations of step β5), k is a natural number between 1 and K, Tk is the first variable with iteration rank k,
${}^tq_k$ is the transpose of the linear regression slope used in step β10
$Y_K$ is the second set of calculation data with iteration rank K,

γ2) determining the set of data of the model ($M_{(px1)}$) and the residue (R) as respectively being equal to

$$M_{(px1)} = \sum_1^K W_k\ {}^tq_k$$

$R = Y_K$
where $W_k$ is the weight coefficient with iteration rank k.

**15.** A use of a method according to one of the preceding claims to determine the basicity index (BN) of a lubricant circulating in a piece of equipment of a ship, in particular in a ship engine.

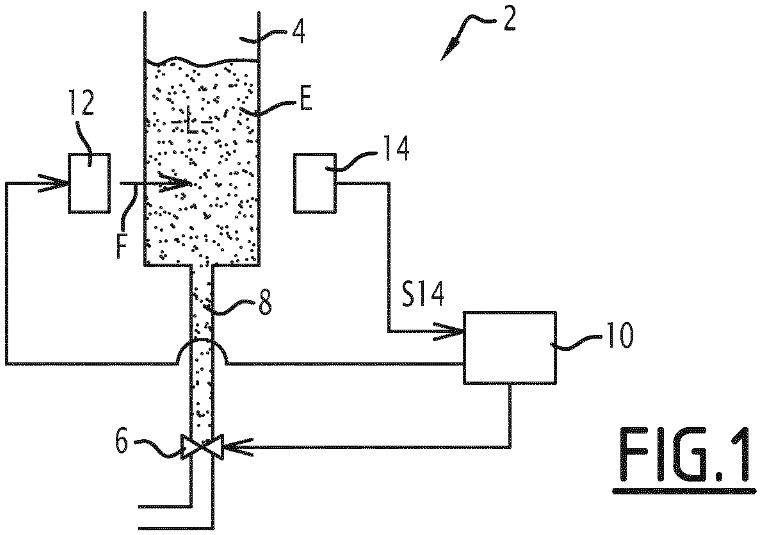


FIG.1

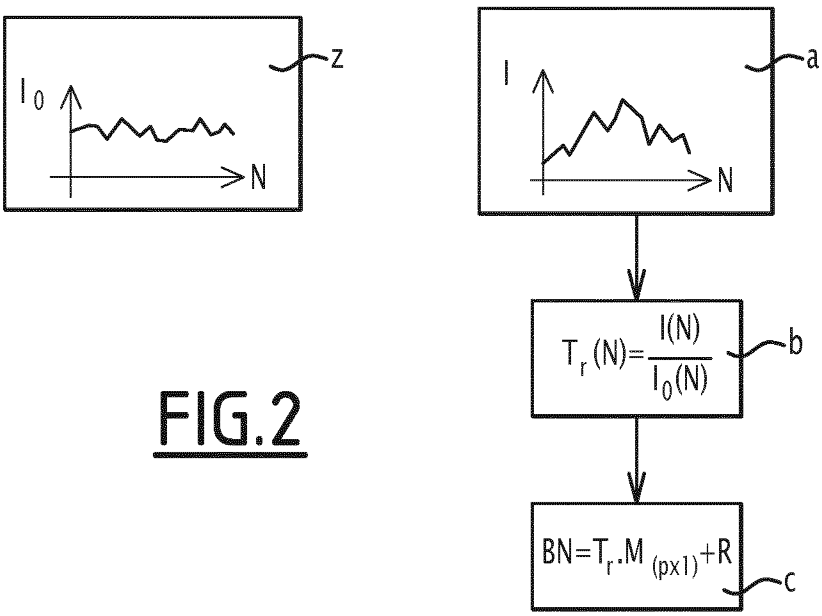


FIG.2

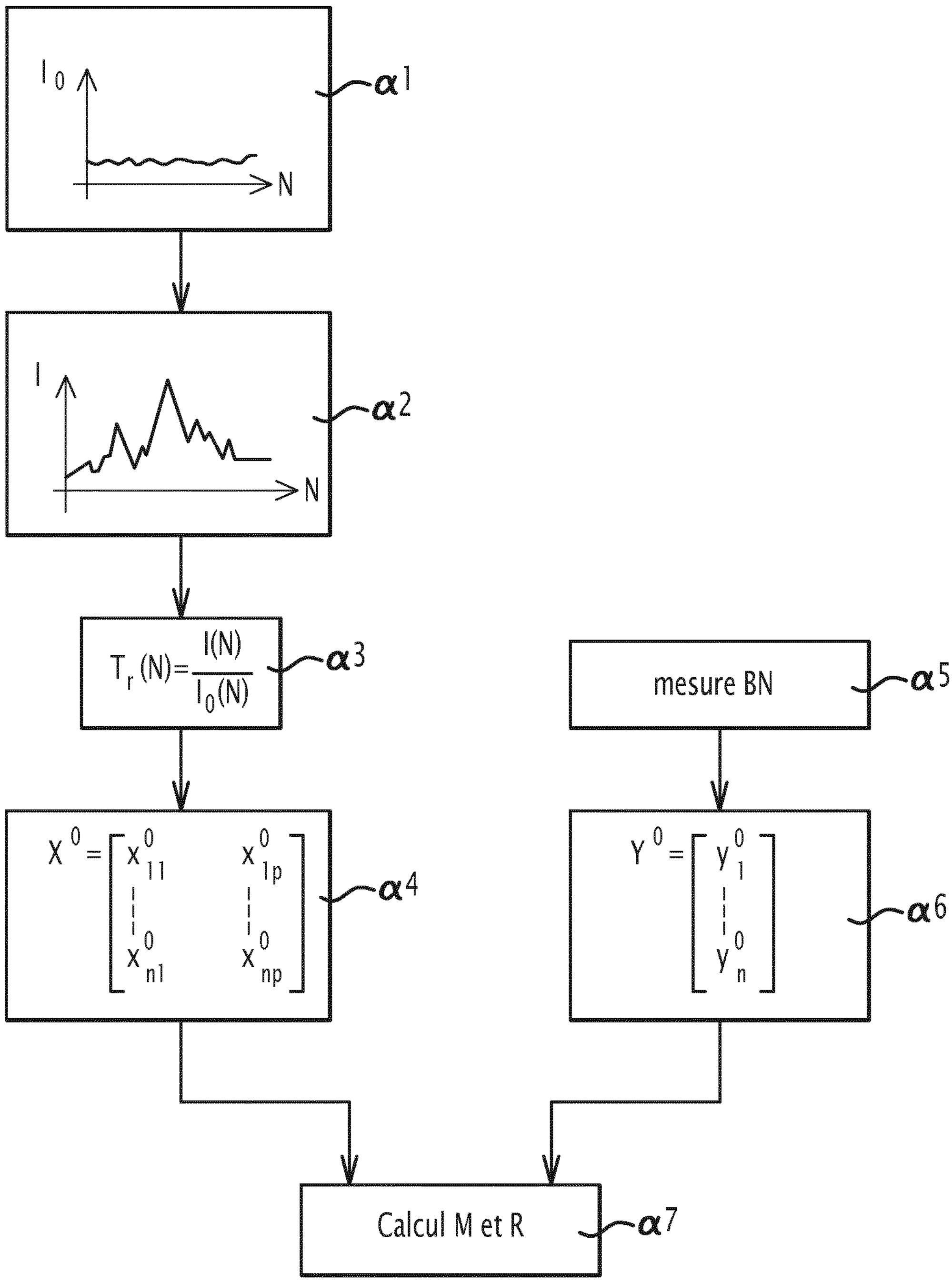

$I_0$
N
α1
I
N
α2
$T_r(N)=\frac{I(N)}{I_0(N)}$
α3
mesure BN
α5
$X^0 = \begin{bmatrix} x^0_{11} & x^0_{1p} \\ x^0_{n1} & x^0_{np} \end{bmatrix}$
α4
$Y^0 = \begin{bmatrix} y^0_1 \\ y^0_n \end{bmatrix}$
α6
Calcul M et R
α7


FIG.3

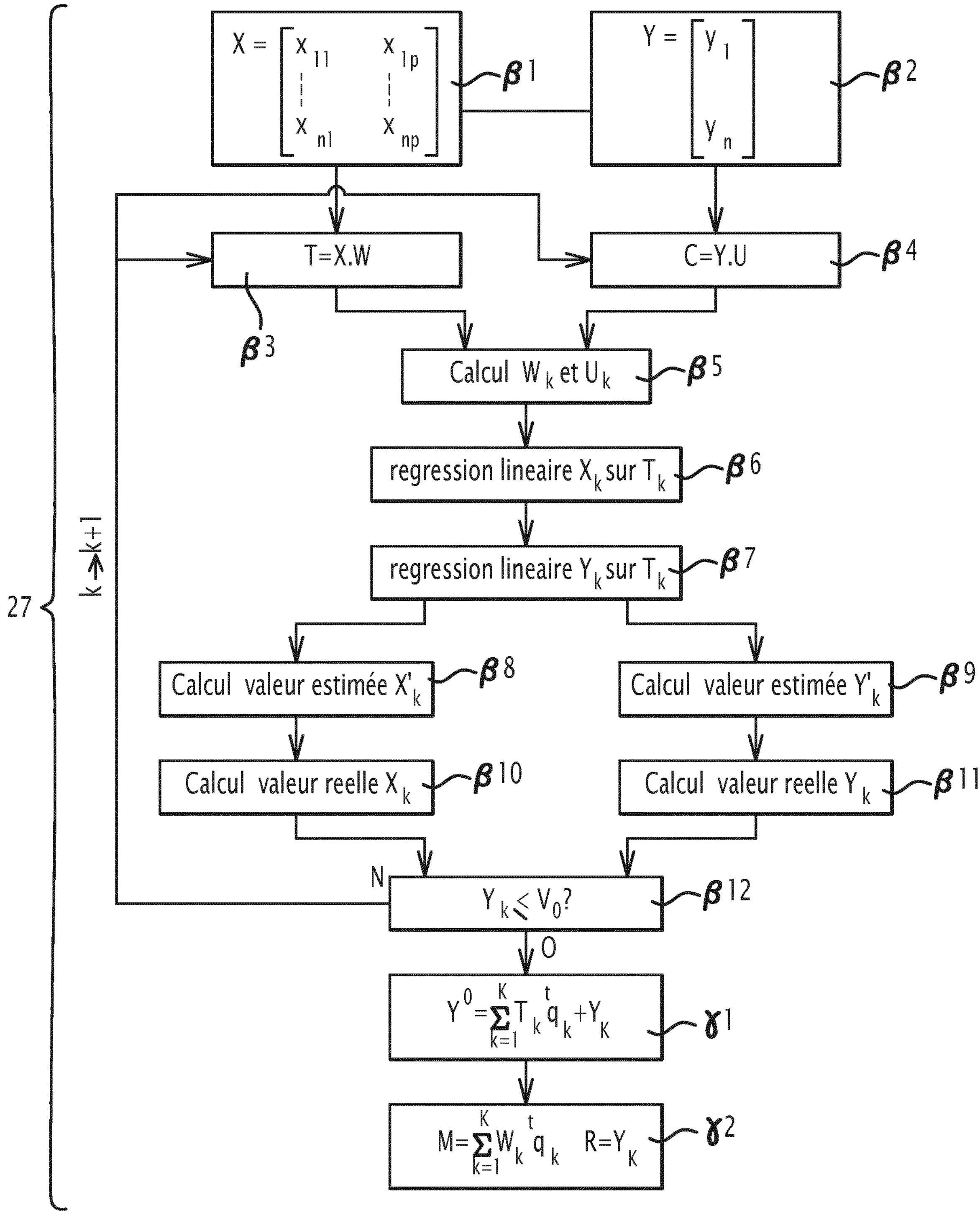
X =
x 11
x 1p
x n1
x np
β1
Y =
y 1
y n
β2
T=X.W
β3
C=Y.U
β4
Calcul W k et U k
β5
regression lineaire X k sur T k
β6
regression lineaire Y k sur T k
β7
Calcul valeur estimée X'k
β8
Calcul valeur estimée Y'k
β9
Calcul valeur reelle X k
β10
Calcul valeur reelle Y k
β11
N
Y k < V0?
β12
O
Y0 = Σ k=1 K T k tq k + Y K
γ1
M = Σ k=1 K W k tq k R = Y K
γ2
k → k+1
27

FIG.4

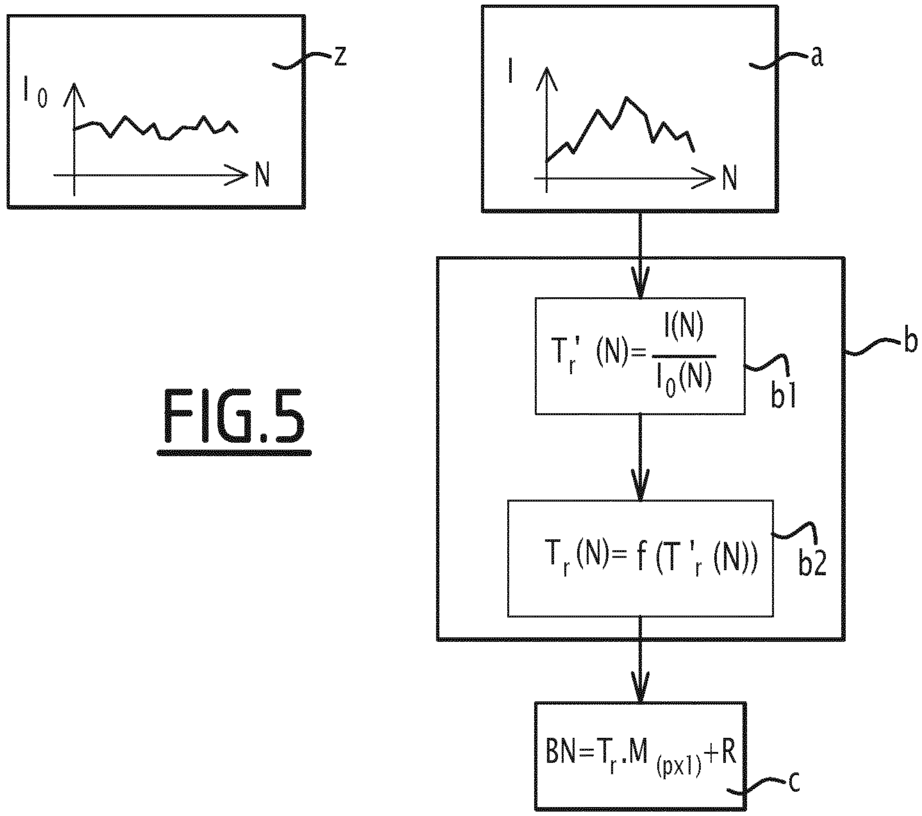


FIG.5

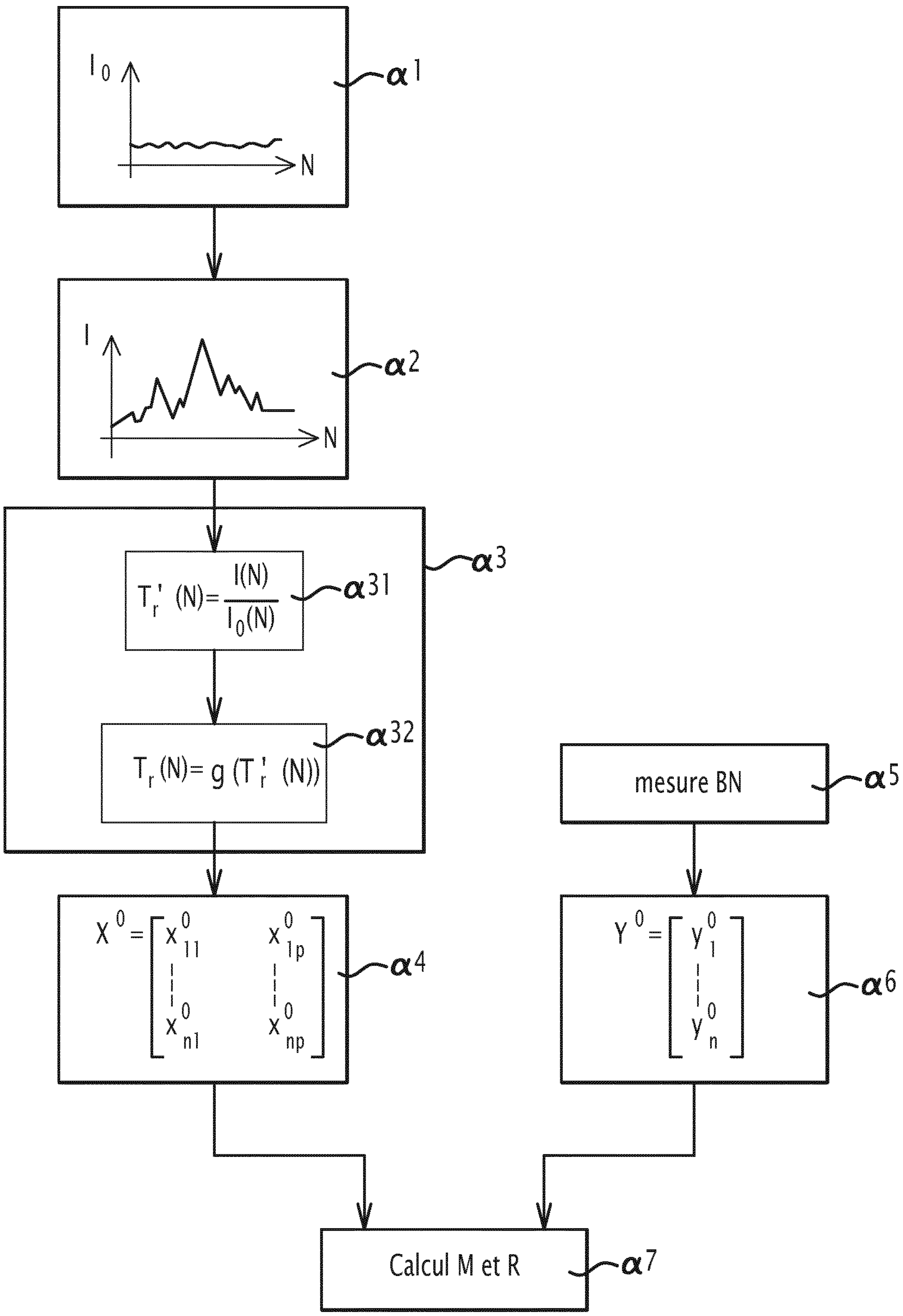

$I_0$
N
α1
I
N
α2
$T_r'(N) = \frac{I(N)}{I_0(N)}$
α31
α3
$T_r(N) = g(T_r'(N))$
α32
mesure BN
α5
$X^0 = \begin{bmatrix} x_{11}^0 & x_{1p}^0 \\ \vdots & \vdots \\ x_{n1}^0 & x_{np}^0 \end{bmatrix}$
α4
$Y^0 = \begin{bmatrix} y_1^0 \\ \vdots \\ y_n^0 \end{bmatrix}$
α6
Calcul M et R
α7


FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03073075 A
- WO 2013186338 A


### Littérature non-brevet citée dans la description


- **BEN MOHAMMADI et al.** A low cost mid-infrared sensor for on line contamination monitoring of lubricating oils in marine engines. *Optical Sensing and Détection Conférence,* 12 Avril 2010
- **AGNAR HÖSKULDSSON.** PLS regression methods. *Journal of chemometrics,* 1988, vol. 2, 221-228